# EUROPEAN PATENT APPLICATION

(11) **EP 0 773 294 A2**
(43) Date of publication of application: **14.05.1997**
(21) Application number: 96115534.8
(22) Date of filing: 30.07.1990
(51) Int. Cl.: C12N 15/16, C07K 14/71, C12N 15/10, C12N 15/85

(54) **Efficient directional genetic cloning system**

(30) Priority: 28.07.1989 US 386053
(62) Divisional of application: 90912327.5
(71) Applicant: THE UNITED STATES OF AMERICA as represented by the Secretary UNITED STATES DEPARTMENT OF COMMERCE, Washington, DC 20231 (US)
(72) Inventor: Miki, Toru, Rockville, MD 20852 (US); Aaronson, Stuart A., Great Falls, VA 22066 (US); Fleming, Timothy, Derwood, MD 20855 (US)
(74) Representative: Killin, Stephen James

(57) **Abstract**

A highly efficient genetic cloning system is disclosed which is particularly useful for cloning cDNA copies of eukaryotic mRNAS and can direct the orientation of inserts in plasmid composite vectors with large cloning capacities. Cleavage of such vector DNA, by the restriction enzyme SfiI, for example, creates two different non-symmetrical 3' extensions at the ends of vector DNA. Using a linker-primer and an adaptor, cDNA is prepared to have two different sticky ends which can be ligated to those of the vector. When the cDNA fragments and the vector DNAs are mixed, both the molecules can assemble without self-circularization due to base-pairing specificity. This system provides (1) high cloning efficiency (10⁷-10⁸ clones/g poly (A)⁺ RNA), (2) low background (more than 90% of the clones contain inserts), (3) directional insertion of cDNA fragments into the vectors, (4) presence of a single insert in each clone, (5) accommodation of long inserts (up to 10kb), (6) a mechanism for rescue of the plasmid part from a λ genome, and (7) a straightforward protocol for library preparation.

## Description

### BACKGROUND OF THE INVENTION

### FIELD OF THE INVENTION

The present invention relates to vectors for molecular cloning of DNA segments, particularly to cloning vectors employing non-symmetrical restriction enzyme recognition sites for insertion of DNA segments in a defined direction relative to vector. This invention also relates to use of such vectors in methods for efficient cloning of genomic DNA segments and of complementary DNA (cDNA) copies of messenger RNA (mRNA) molecules from eukaryotic genes, and to the manufacture and use of novel products related to these vectors and methods.

### BACKGROUND OF THE INVENTION

The development of DNA cloning techniques for complementary DNA (cDNA) copies of messenger RNA (mRNA) molecules has been of great value in the study of eukaryotic genes. In many cases, the amount of a given mRNA for which cDNA clones are desired is limited by the availability of appropriate tissue sources and/or a low concentration of that specific mRNA in those sources. Therefore, readily obtainable sources may provide only a few copies of a given mRNA molecule from which cDNA clones might be produced.

The requirements for any efficient method for cDNA cloning may be generally summarized as follows: first, full-length double-stranded cDNAs must be produced from the mRNA with high yield; the ends of the resulting DNA fragments must be made capable of being joined efficiently to the vector DNA by enzymatic ligation; production of undesirable ligation byproducts must be minimized; and, preferably, insertion of the cDNA into the vector DNA should provide expression of the cDNA to facilitate detection of the desired clone by means of the product.

Production of the protein product may be necessary for detecting a gene when no nucleic acid probes for the desired gene are available. More generally, such expression of the protein is desirable because, in terms of copy number, the protein provides a molecular signal that is greatly amplified in relation to the DNA molecules of the cloned gene inside the host cell.

As it is difficult to achieve high efficiency of conversion of mRNA molecules into full-length cDNA clones, especially when the mRNA of interest is relatively long, several refinements in cDNA cloning strategy have been made. Among them, the Okayama-Berg method significantly improved the efficiency of full-length cDNA cloning.

The Okayama-Berg approach has several advantages over previous, conventional methods for cloning cDNAs. The following section is intended to highlight these advantages in relation to the main steps of this complicated method. For a more complete and detailed description of the method, see the original publication [Okayama, H. and Berg, P. (1982) Mol. Cell. Biol. *2*, 161-170], which is hereby incorporated herein by reference.

The main advantages of the Okayama-Berg method for cDNA clone relate to the fact that as part of the processing needed to form mRNAs, transcripts of eukaryotic genes undergo enzymatic addition of multiple adenosine residues at the 3' end, thereby acquiring what is known as a "poly(A) tail". In the present context, the term mRNA encompasses any RNA species from any source, natural or synthetic, having a 3' poly(A) tail comprising two or more adenosine residues.

In the original Okayama-Berg approach, synthesis of the first DNA strand from the mRNA template is initiated by annealing the 3' poly(A) of the eukaryotic mRNA to an oligo(dT) primer which forms an extension of one end of a DNA strand of the cloning plasmid. First strand cDNA synthesis by this "plasmid-priming" method directs the orientation of the sequence within the cDNA into a unique relationship with the sequence in the plasmid; hence, this approach has been called "directional" cloning. Directional cloning ensures that every cDNA clone that is formed will be correctly oriented for a promoter provided in the cloning plasmid (an SV40 promoter in the original Okayama-Berg system) to drive transcription of the proper cDNA strand to produce RNA with the correct sense for translation into the protein encoded by the original mRNA template.

To provide high efficiency of ligation in cloning DNA segments in general, restriction nucleases are utilized to produce short single-stranded ends on the DNA that are complementary in base sequence to any other DNA end produced by the same enzyme. Accordingly, these single-stranded ends can anneal together by forming specific DNA base pairs, or, in the vernacular, they are "sticky". This annealing greatly enhances the rate of joining DNA segments by enzymatic ligation and further provides a means for selectively joining ends of segments treated with the same enzyme.

In the original Okayama-Berg method, after synthesis of the first cDNA strand, an oligo(dG) tail is attached enzymatically to the free end of the plasmid-primed cDNA, and then the plasmid is cleaved by a restriction enzyme (*Hind*III) to produce a sticky end on the plasmid opposite to the end where the cDNA is attached. A short DNA fragment ("linker"), which contains the SV40 promoter and has a cleaved *Hind*III site on one end and oligo(dC) on the other, is then attached to the cDNA-plasmid molecule by ligation, to circularize the molecule.

In other, more conventional methods a (synthetic) linker may also be used to clone cDNAs, but it is attached after second strand DNA synthesis and further enzymatic repair which is necessary to form perfectly matched strands (i.e., a "flush" or "blunt" end). To protect internal restriction sites of the double-stranded cDNA from cleavage by the restriction enzyme required to allow ligation of the vector and linker, prior to addition of the linker, the cDNA is methylated with the appropriate DNA modification system associated with the given restriction enzyme. However, such protection may not be absolute; thus, internal sites may be cleaved at some frequency due to an incomplete methylation reaction. In contrast, in the original Okayama-Berg method, this problem of internal cleavage of cDNAs is obviated by cleavage of *Hind*III sites on the vector when the cDNA is represented as an RNA:DNA hybrid that resists restriction.

The Okayama-Berg approach provides yet another advantage over previous methods in which both ends of separately synthesized cDNAs are ligated to the vector ends at the same time, namely that according to Okayama-Berg, the necessary circularization of the vector DNA with the cDNA attached at one end is relatively efficient via the linker because only one juncture between the cDNA and vector molecules remains to undergo ligation.

Furthermore, the overall Okayama-Berg approach offers additional advantages over previous methods. Following circularization, a process called "RNA nick translation" using DNA polymerase I and RNase H is used which facilitates complete synthesis of the second strand along the entire first strand. This process overcomes the inherently low processivity of DNA polymerase I by using multiple sites for priming of second strand DNA synthesis with DNA primer fragments having random sequences.

Finally, since the Okayama-Berg vector has already been joined to the cDNA when the second strand is synthesized, truncation of cDNA molecules close to the 3' end of the cDNA generally does not occur, in contrast to other methods in which the second strand is completed while the 3' end of the first strand is free and, therefore, more susceptible to damage from nuclease activities.

Cloning vectors based on bacteriophage λ are also known. The second strand synthesis reaction of the Okayama-Berg method has also been utilized in a simpler cloning procedure [Gubler, U. and Hoffman, B. J. (1983) Gene *25*, 253-269], allowing cDNA cloning in such λ vectors [Huynh, T.V., Young, R. A. and Davis, R.W. (1985) *in* DNA Cloning, A Practical Approach, *ed*. Glover, D. (IRL, Oxford), Vol. I, pp. 49-78]. This λ-based cDNA cloning method has been widely used, mainly due to the high efficiency of transmission of recombinant DNA into cells by means of infectious phage particles, which are produced with *in vitro* DNA "packaging" systems. λ phage cloning systems also offer convenient clone screening capabilities due to tolerance of a high density of λ plaques on test plates to be screened, compared with most plasmid systems which permit only lower densities of bacterial host colonies.

Early λ systems for cDNA cloning, however, while retaining the second strand synthesis strategy of the original Okayama-Berg plasmid method, lack some of its other advantages. For example, directional cloning is not possible in those original λ systems. In addition, multiple inserts and truncated cDNAs are frequently obtained. Further, despite the high packaging efficiency for native λ DNA molecules, the packaging efficiency of recombinant DNA molecules that are produced by cleavage of intact linear λ molecules and ligation with cDNA fragments is usually low compared to that of intact λ DNA.

Recently, directional cloning capabilities have been introduced into various λ vectors. For example, one such directional λ vector employs a site for insertion of DNA segments that comprises two different restriction enzyme cleavage sites [Meissner, P. S., et al. (1987) Proc. Nat. Acad. Sci. USA, *84*, 4171-4175]. The cDNA molecules are primed with oligo(dT), made double-stranded, and then methylated with the enzymes needed for protection against internal cleavage by both of the nucleases used in the DNA insertion site of the vector. A linker segment containing a cleavage site for only one of the nucleases of the insertion site is added to both ends of the cDNA. The combination of the last two A:T base pairs on the 3' end of the cDNA with the sequences at one end of the linker, however, creates a cut site for the other of the two nucleases of the insertion site. Thus, after restriction with both nucleases of the insertion site, the individual cDNA segments can ligate into the vector only in a single direction with respect to the two different cleavage sites in the vector.

Various general disadvantages of this particular approach for cDNA cloning in λ phage, compared to the Okayama-Berg plasmid method, have been described above in relation to other systems; and other problems specific to this approach have been noted [Meissner, P. S., *et al*. (1987), *supra*]. Nevertheless, it was reported that one cDNA library constructed by this method, starting from 5 µg of mRNA, contained about 2 x 10⁸ clones with 8 of 10 having cDNA inserts (i.e., the reported cloning efficiency was about 3 x 10⁷ recombinants per µg of poly(A)+ RNA).

Directional cloning in other λ phage vectors has also been reported [Palazzolo, M. J. and Meyerowitz, E. M. (1987) Gene 52, 197-206]. [These vectors are known as λSWAJ or λGEM, certain variants of which (LambdaGEM™2 and LambdaGEM™4) are commercially available from Promega Corporation of Madison, Wisconsin. The λGEM type of vectors are also examples of a composite vector comprising both a λ phage genome and an embedded plasmid (GEM)]. The directional cloning scheme in these λ vectors utilizes two different restriction enzyme cleavage sites at the site for insertion of DNA. Thus, for example, to attach the end of a cDNA corresponding to the poly(A) end of the mRNA to a particular end of the cleaved vector DNA that has a sticky end for the restriction enzyme *Sac*I, a synthetic DNA "linker-primer" segment is used which combines a single-stranded oligo(dT) primer with a restriction site for the enzyme *Sac*I. After second strand synthesis, a linker segment with the site of a second restriction enzyme is ligated to the other end of the cDNA, which is then restricted with both enzymes of the insertion site of the vector, according to much the same strategy as described for the previous example of a directional λ phage vector.

This particular approach for directional cloning in a λ vector, however, cannot be used to obtain full-length cDNAs of certain mRNAs because it requires cleavage of the cDNA molecules by the restriction enzyme *Sac*I and a second enzyme (e.g., *Xba*I) without first protecting the internal sites for these enzymes by appropriate methylation. [In an alternative version of the scheme reported by Palazzolo and Meyerowitz, *supra*, the *Xba*I enzyme was replaced by *Eco*RI and the cDNA was methylated to protect against only this one enzyme.] Sites for these particular enzymes occur frequently by chance in natural nucleotide sequences. Thus, restriction of cDNAs with enzymes like these, as taught in this approach, causes truncation of cDNA inserts with internal *Sac*I (and/or *Xba*I) sites. In relation to cloning efficiency, it may be noted that this publication described a single cDNA library constructed by this method, starting from 1 µg of poly(A)⁺ RNA, that contained about 1.6 x 10⁶ clones with cDNA inserts.

In addition to the publications on directional cloning systems described above, there is a report which describes a non-directional plasmid-based system that uses an efficient oligonucleotide-based strategy to promote cDNA insertion into the vector [Aruffo, A. and Seed, B. (1987) Proc. Nat. Acad. Sci. USA *84*, 8573-8577]. This method uses synthetic DNA adaptors that encode a recognition site for a particular restriction enzyme, *Bst*XI, which has a variable recognition sequence, as illustrated below: 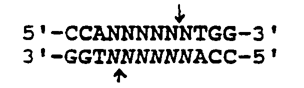
5'-CCANNNNNNNTGG-3'
3'-GGTNNNNNNNACC-5'
 where A, T, G and C indicate nucleotides having the DNA bases adenine, thymine, guanine, and cytosine, respectively (for which the pairs A:T and G:C are complementary), and N and *N* represent bases that are included within the recognition site sequence but that can be any of the usual DNA bases, provided only, of course, that each N and the corresponding *N* on the opposite DNA strand be complementary. The arrows (↓ and ↑) indicate the cleavage sites on the upper and lower DNA strands, respectively. Accordingly, cleavage of the *Bst*XI site creates a 4-base single-stranded extension (sticky end) on the 3' end that varies from site to site.

The report above discloses a plasmid vector with a site for insertion of DNA segments in which two identical *Bst*XI sites were placed in inverted orientation with respect to each other and were separated by a short replaceable segment of DNA. Inversion of a DNA sequence consists of representing the base sequence of each strand, conventionally expressed in the 5' to 3' direction of the polynucleotide backbone, in a DNA strand with the same base sequence presented in the 3' to 5' direction (e.g, inversion of the DNA sequence 5'-ACTG-3' produces the DNA sequence 3'-ACTG-5' or, in the conventional 5' to 3' format, 5'-GTCA-3'.

With the particular *Bst*XI recognition sequence that was employed in this vector, the 4-base single-stranded ends of the inverted sites created on the two ends of the vector DNA by restriction with the *Bst*XI enzyme were not able to anneal with one another. This situation is illustrated below, where two identical sites, one inverted relative to the other and separated by an unspecified sequence (N...N), are shown; the **sticky ends of the vector** produced by cleavage with the *Bst*XI enzyme are **shown in bold print**: 
5'-(vector)CCANTGTGNTGG(N…N)CCANCACANTGG(vector)-3'
3'-(vector)GGTNACACNACC(N…N)GGTNGTGTNACC(vector)-5'
 (Note that the reference does not specify the entire *Bst*XI recognition sequence that was used; only the sequence of the sticky end is clearly defined, as indicated below by inclusion of the N symbol where necessary).

Inspection of these single-stranded end sequences on this plasmid vector reveals that they are identical, due to the inversion of one of the sites relative to the other. Thus, the ends of the vector with inverted and non-inverted copies of this particular *Bst*XI restriction site sequence cannot anneal with each other. Similarly, the restricted ends of the spacer DNA segment between these two sites will be identical. Accordingly, to clone cDNA segments in this vector, a synthetic adaptor was attached to each end at the double-stranded stage, by blunt end ligation, giving them the same termini as the replaceable segment that was removed from the vector with *Bst*XI. The specific adaptor used in the above report comprises the following oligonucleotide sequences: 
5'-CTTTAGAGCACA-3'
3'-GAAATCTC-5'.
 Obviously, addition of this single adaptor to both ends of the cDNA segments would provide those segments with ends (**in bold type**) that could anneal and subsequently ligate efficiently to both identical vector ends.

Thus, Aruffo and Seed, 1987, *supra*, discloses a method using this particular *Bst*XI recognition site sequence, whereby neither the cDNA (with attached adaptors) nor the isolated vector DNA (after being freed from the replaceable segment after cleavage with *Bst*XI) was able to ligate to itself. This work, however, neither teaches nor suggests general requirements for a *Bst*XI recognition sequence, or for those of other restriction enzymes, to be usable in this cloning approach.

Further, as these workers pointed out, their strategy did *not* provide a directional cloning capability. After first alleging that such directional capability was not needed, they admitted that, nonetheless, they had devoted considerable unsuccessful efforts to developing an alternative means of producing mRNA from every cDNA clone, namely a bidirectional transcription capability whereby both strands of an inserted cDNA would be transcribed. They concluded that this goal cannot be easily attained, at least not in their cloning host system. The authors stated, moreover, that they could obtain cloning efficiencies with their plasmid that were between 0.5 and 2 x 10⁶ recombinants per µg of mRNA, which were said to compare favorably with those described for certain cloning systems based on phage λ. In the only example of a cDNA library described in this reference, however, the yield of cDNA clones obtained by this method was actually stated to be only ≈ 3 x 10⁵ recombinants from 0.8 µg poly(A)-containing RNA (i.e., less than 0.4 x 10⁶ recombinants per µg poly(A)-containing RNA).

Thus, there has been a continuing need for methods and vectors which would provide a higher yield of cDNA clones from limited amounts of eukaryotic mRNAs while also providing an improved means of directing orientation of inserted cDNA fragments within vector DNAs.

### SUMMARY OF THE INVENTION

The present invention contemplates the application of methods of recombinant DNA technology to fulfill the above needs for increased efficiencies in DNA cloning systems and, in particular, to develop new means for directional insertion of cDNA fragments into cloning vectors.

More specifically, it is an object of the present invention to provide means for directing assembly of insert DNAs into vector DNAs to form a unique, predetermined recombinant structure having the desired number and orientation of each needed DNA fragment, so that the number of resulting clones containing single inserts, as well as the probability of obtaining a full-length clone from each mRNA molecule, are enhanced.

Further, it is an object of this invention to provide a cDNA cloning system which combines the features of this highly efficient cloning strategy with advantageous features of λ phage vectors to overcome limitations of the presently available λ cloning systems.

Accordingly, the present invention relates to highly efficient means for inserting DNA segments into cloning vectors in a defined orientation, and a method for using such means that is referred to herein as the "automatic directional cloning (ADC)" method. Novel DNA vectors and DNA segments are also included.

Appreciation of the operation and advantages of this invention requires further analysis of the problems in the prior approaches. The understanding of these problems by the present inventors lead to development of this invention.

The present invention has been developed in light of recognition by these inventors of major sources of the limitations on cloning efficiency with the present systems designed for directional cloning, that they all employ restriction enzymes with recognition site sequences having one or both of the following disadvantages: they are either too short or they have a particular type of symmetry called "dyad" symmetry.

As noted above, present λ phage vectors for directional cloning of cDNAs suffer inefficiencies due in part to their use of restriction enzymes with recognition sequences that occur frequently in natural DNA sequences. Some problems relating to this issue might be solved by choosing a restriction enzyme with an infrequently occurring site (i.e., a longer recognition sequence which, by chance, would occur less frequently in random natural DNA sequences).

However, even when modified to utilize an infrequently cutting restriction enzyme, the present implementations of directional cloning in λ have a drawback that is common to any cloning scheme using restriction enzymes with recognition sequences having dyad symmetry of the sticky ends produced by cleavage with the enzyme. Typical restriction enzymes with recognition site sequences having dyad symmetry make staggered cuts in the two opposing DNA strands at symmetrical points surrounding the center of a dyad pattern. Cleavage by this type of enzyme produces short single-stranded ends which are complementary in base sequence to those of any other DNA fragment produced by cleavage with the same enzyme.

For example, the recognition site of the commonly used restriction enzyme, *Eco*RI, consists of the following complementary sequences which when cleaved by the enzyme, produce the 4-base extension of the 5' end of the DNA containing the dyad "TTAA" (**shown in bold face type**): 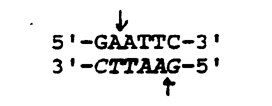 where A, T, G and C indicate DNA bases, as described above for the *Bst*XI site. Inspection of this *Eco*RI sticky end sequence readily reveals that inversion of this sequence produces its complement, namely "AATT". Thus, any DNA end produced by *Eco*RI can anneal to any other such DNA end; and, therefore, any *Eco*RI sticky end can also be ligated efficiently to any other such end. Similarly, all DNA ends which are produced by *any* one restriction enzyme that generates sticky ends characterized by dyad symmetry are in the case of each sticky end sequence readily ligatable one to another. [Hereinafter, such "self-ligatable" single-stranded ends of DNA that are producible by a restriction enzyme will be simply referred to as "symmetrical ends", and the enzymes that produce them, as "symmetrical restriction enzymes".]

In light of this symmetrical nature of many restriction enzyme recognition sites, one of the major problems with existing directional λ vectors can be more fully appreciated. When the two end fragments of cleaved λ DNA (i.e., the so-called λ "arms") are ligated with cDNA fragments, several products are produced, only some of which constitute the desired infectious DNA molecules containing cDNA inserts. For instance, consider the simplest case, when the ends on both the cDNA and on each of the "left" and "right" λ arms (as the two λ DNA arms have been designated in a genetic mapping convention) have been cut by the same symmetrical restriction enzyme. Here, linear structures other than those with the desired order (i.e., "left arm-cDNA insert-right arm") may form in significant amounts during ligation with cDNA fragments; and the cDNAs trapped in these other, nonviable structures cannot produce phage clones. These undesirable ligation byproducts may include self-ligation products of the two ends of individual vector or cDNA segments, consisting of circular DNAs. Ligation products in this instance may also comprise vector-cDNA combinations containing multiple inserts, which, even if viable, may create problems in expression or identification of original mRNA structure.

On the other hand, when each end of the vector and insert cDNA molecule are ultimately produced by two different symmetrical restriction enzymes, as in the present directional λ systems, these ends are then physically distinguishable in relation to the polarity of the encoded genetic information in each DNA segment, i.e., the matching of complementary sticky ends on vector DNAs and cDNAs results in the desired directional cloning of the cDNA insert relative to functional sequences in the vector (e.g., a promoter). Further, circularization due to self-ligation of cDNAs or vectors without inserts is eliminated by the use of two different symmetrical restriction enzymes.

Other undesirable ligation byproducts remain, however, in the usual two enzyme approach for directional cloning using symmetrical enzymes. Some of these are dimers of vector or cDNAs, which may be designated, for example, as "tail-to-tail" or "head-to-head" dimers. Thus, even when vector and cDNAs are made by cutting with two different symmetrical enzymes, head-to-head and tail-to-tail dimers are not eliminated, although the population of desired molecules is significantly higher.

In contrast to existing systems based on λ phage, the automatic directional cloning method does not permit cDNA or vector fragments to ligate to each other, ensuring the presence of a single insert in each clone, as well as higher cloning efficiencies and lower backgrounds of clones that do not contain cDNA inserts.

To accomplish these goals, the present invention contemplates use of restriction enzymes which produce single-stranded ends that do not exhibit dyad symmetry (hereinafter referred to as "non-symmetrical ends" and correspondingly, non-symmetrical recognition site sequences and nzymes). Although certain preferred embodiments of the present invention employ derivatives of bacteriophage λ as the vector, which further comprise embedded plasmid genomes, this invention can be practiced with any self-replicating DNA molecule (i.e., a "replicon") serving as the vector for DNA cloning in any host in which the selected replicon can be replicated.

Work cited in the Background above describes a plasmid-based system that advantageously employs two *identical Bst*XI recognition site sequences, albeit in two different orientations. This single recognition sequence is non-symmetrical according to the definition in the present disclosure, although the reference does not describe the *Bst*XI sequence in such terms or otherwise characterize this sequence as such. The present invention is clearly distinguishable from this previous approach, as described below.

Use of *Bst*XI sites is not readily applicable to the λ system, due to the existence of multiple *Bst*XI recognition sites in the λ phage genome, owing to the number of base pairs in the variable recognition sequence that are not allowed to vary (i.e., the "invariable base pairs" being only six).

Accordingly, in one aspect the present invention relates to a genetic cloning vector comprising at least one replicon, and a site for inserting DNA segments to be cloned that includes at least two non-symmetrical restriction enzyme recognition sequences that are identical, where the first of these identical recognition sequences is in the inverted orientation with respect to a second identical sequence; and, in addition, the identical restriction enzyme recognition sequences include greater than six positions having invariable DNA base pairs. Recognition site sequences of the enzyme *Sfi*I, for example, fulfill both the length and asymmetry requirements for this aspect of the invention, as will become evident below.

On the other hand, in plasmid systems or other replicons lacking *Bst*XI sites, either naturally or due to genetic engineering, two *Bst*XI site sequences that are in the same instance non-symmetrical and *nonidentical* can be advantageously employed for efficient cloning of DNA segments according to the present invention.

More generally, this aspect of this invention may be practiced with any two non-symmetrical restriction enzyme recognition sequences that are not identical (recognitions site sequences of the enzyme *Sfi*I, for instance).

Accordingly, the present invention also relates to a genetic cloning vector comprising at least one replicon, and a site for inserting DNA segments to be cloned that includes at least two non-symmetrical restriction enzyme recognition sequences that are nonidentical. In this aspect of the invention, two of the non-symmetrical restriction enzyme recognition sequences can be selected advantageously to be cleavable by a single restriction enzyme, for example, *Bst*XI or, alternatively, *Sfi*I; or each of two nonidentical restriction enzyme recognition site sequences may be selected to be cleavable by a different enzyme. Preferably, at least one of the non-symmetrical recognition sequences includes greater than six positions having invariable DNA base pairs; and most preferably, two nonidentical recognition sequences include greater than six positions having invariable DNA base pairs, as typified by *Sfi*I recognition site sequences.

The present invention further relates to a vector, as described above, in which the replicon comprises a form of bacteriophage λ.

The vector may advantageously further comprise regulatory elements located in relation to the site for insertion of DNA segments such that, when a DNA segment is inserted into this site, at least a portion of the sequences of the DNA segment is transcribed. This portion may be derived from either one of the strands of the inserted double-stranded DNA segment, or from both of these strands.

In one major embodiment of this aspect of this invention, these regulatory elements in the vector consist of promoters that entirely originate from bacteriophage. By the phrase "originate from" it is meant that the regulatory element (e.g., promoter) is encoded in the genome of the instant organism or virus (e.g., bacteriophage) as it occurs in nature. It should be noted here that it is well known that, generally, promoters that originate from bacteriophage are not able to initiate transcription in eukaryotic hosts. This particular embodiment of the present invention is exemplified by two λ-plasmid composite vectors, LambdaGEM™11 and LambdaGEM™12, which are commercially available from Promega Corporation of Madison, Wisconsin.

According to available information at the time of the present disclosure, these particular LambdaGEM™ vectors apparently were first disclosed in the 1988/1989 Catalogue and Applications Guide for Biological Research Products published and distributed by Promega Corporation in August of 1988, the entirety of which is hereby incorporated herein by reference. The following excerpts of that catalog describe these particular vectors and some of their various uses, particularly those relating to transcription from bacteriophage promoters.

### Section 11, page 5:

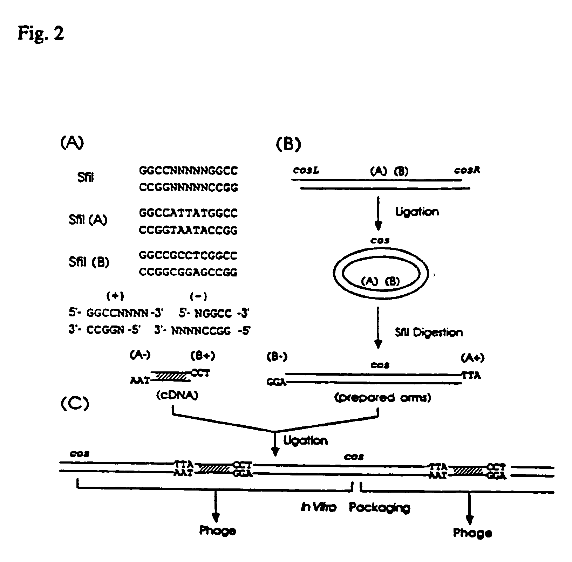

The LambdaGEM-11 vector is a multi-functional genomic cloning vector designed for high resolution mapping of recombinant inserts, simplified genomic library construction, ultra-low background of non-recombinants, and rapid genomic walking. This lambda replacement-type cloning vehicle contains the following features (Figure 2 [not shown]): dual opposed bacteriophage T7 and SP6 RNA polymerase promoters, flanking asymmetric *Sfi*I restriction sites, and a multiple cloning site with strategically positioned *Xho*I and *Bam*HI restriction sites. The LambdaGEM-11 vector also contains unique sites for *Sac*I, *Avr*II, *Eco*RI, and *Xba*I. Because it is a derivative of EMBL3 (1), DNA fragments ranging from 9-23kb can be cloned in the LambdaGEM-11 vector and the *Spi* phenotypic selection against non-recombinants is available. The vector was designed to make use of the *Sfi*I recognition sites flanking the promoters for the high resolution restriction mapping of insert DNA using the *Sfi* linker mapping system (Sec. 11, pg. 8).

The T7/SP6 phage promoters simplify chromosomal "walking", as RNA probes synthesized from the extremities of the cloned insert can be used to search a library for overlapping sequences in either direction. In addition, the nucleotide sequence of the end of an insert cloned in the LambdaGEM-11 vector can be obtained directly form the phage template by hybridizing an SP6 or T7 oligonucleotide primer, followed by a chain termination sequencing reaction (2,3).

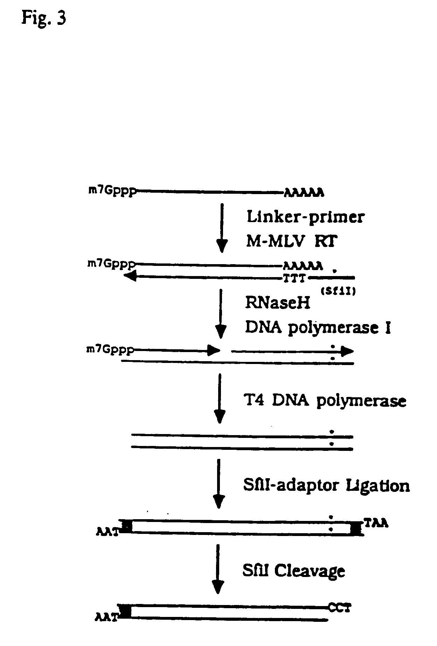

Two cloning strategies for genomic library construction, using DNA partially digested with *Mbo*I or *Sau3A*I, are available with the LambdaGEM-11 dephosphorylated *Bam*HI arms. A new cloning strategy (4) relies on the exclusive specificity with which partially filled-in *Xho*I LambdaGEM-11 arms (*Xho*I half-site arms) can be combined with partially filled-in *Sau3A*I digested genomic DNA. The only ligation products possible are single copies of genomic inserts with appropriate arms, since the partial fill-in prevents self-ligation reactions of vector arms, central stuffer, and genomic fragments. This method also makes genomic DNA fractionation unnecessary, is very rapid (Figure 3 [not shown]), and requires small amounts of starting material. The *Xho*I and *Bam*HI sites in the LambdaGEM-11 vector are strategically positioned 6 and 11 bases, respectively, from the transcription initiation site of either promoter.

As measured by *in vitro* packaging, recombinant efficiencies of 3 x 10⁷ pfu/µg DNA have been achieved using a test insert ligated to LambdaGEM-11 dephosphorylated *Bam*HI or *Xho*I half-site arms using Packagene® lambda packaging extracts. The background for self-ligated arms alone is typically <100 pfu/µg DNA in either case. This ultra-low background level of non-recombinant vector DNA has three important advantages: it eliminates the need for the *Spi* genetic selection against the parental vector, which is known to result in biased libraries (5), non-productive ligation events are minimal, thereby resulting in larger genomic libraries, and fewer filters need be processed for screening a library. For detailed protocols describing the use of this vector, see Sec. 11, pg. 12.

### Section 11, page 6:

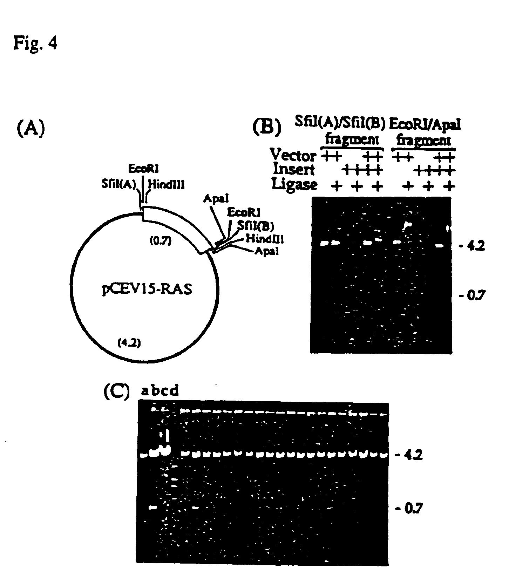

The LambdaGEM-12 vector is a multi-functional genomic cloning vector designed for high resolution restriction mapping of recombinant inserts, simplified genomic library construction, ultra-low background of non-recombinants, and rapid genomic walking. This lambda replacement-type cloning vehicle contains the following features (Figure 4 [not shown]): dual opposed bacteriophage T7 and SP6 RNA polymerase promoters, RNA polymerase promoters [*sic*], flanking asymmetric *Sfi*I restriction sites, and a multiple cloning site with strategically positioned *Not*I and *Bam*HI restriction sites. The LambdaGEM-12 vector also contains unique sites for *Sac*I, *Eco*RI, *Xho*I, and *Xba*I. Because it is a derivative of EMBL3(1), DNA fragments ranging from 9-23kb can be cloned in the LambdaGEM-12 vector and the *Spi* phenotypic selection against non-recombinants is available.

Accordingly, the present invention relates to a vector that is either LambdaGEM 11 or LambdaGEM 12. Further details of the use of these vectors for restriction mapping of inserted DNA segments, according to the *Sfi* Linker Mapping System mentioned above, are extracted from the Promega catalog below.

### Section 11, page 8:

The multi-functional LambdaGEM -11 and LambdaGEM -12 genomic cloning vectors have been engineered specifically for high resolution restriction mapping of recombinant inserts. The vectors, derivatives of EMBL3, possess *Sfi*I restriction sites flanking bacteriophage T7/SP6 RNA polymerase promoters and a multiple cloning region (Sec. 11, pg. 5). The flanking *Sfi*I restriction sites allow most inserts to be excised as a single fragment, since this 8-base recognition sequence occurs infrequently in genomic DNA (in theory, once every 65,536bp).

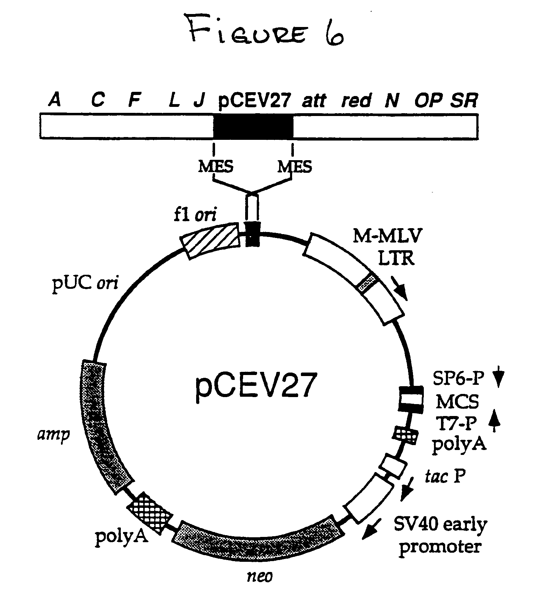

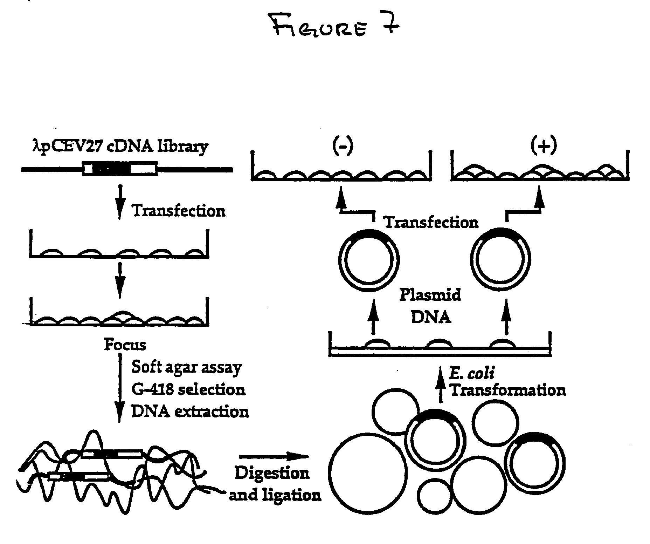

*Sfi*I recognizes the interrupted palindrome GGCCNNNN/NGGCC and cleaves within the central unspecified sequence, leaving a 3-base 3' overhang. The nucleotide sequence of the central region which becomes the overhanging termini thus may contain any of the four possible bases. The flanking *Sfi*I sites in the vectors have been designed in an asymmetric fashion, so that the site on the left is distinct from the site on the right. Therefore, radiolabeled linkers complementary to either the left or right *Sfi*I termini can be ligated separately to the *Sfi*I excised genomic DNA. Once the insert has been asymmetrically labeled, a high resolution restriction map can be determined by partial digestion with a frequent cutting restriction endonuclease such as *Sau3A*I followed by gel electrophoresis and autoradiography (Figure 6 [not shown]).

The mapping resolution of this method is an order of magnitude greater than conventional cos site oligo labeling, since only the ends of the centrally located insert are labeled instead of the ends of the 20kb and 9kb arms of the vector. The variable results generated from inaccurate size estimates of large restriction size fragments, as well as anomalous bands which result from the fusion of the insert with a vector fragment, are eliminated with this system. For a detailed protocol describing the use of this system, see Sec. 11, pg. 14.

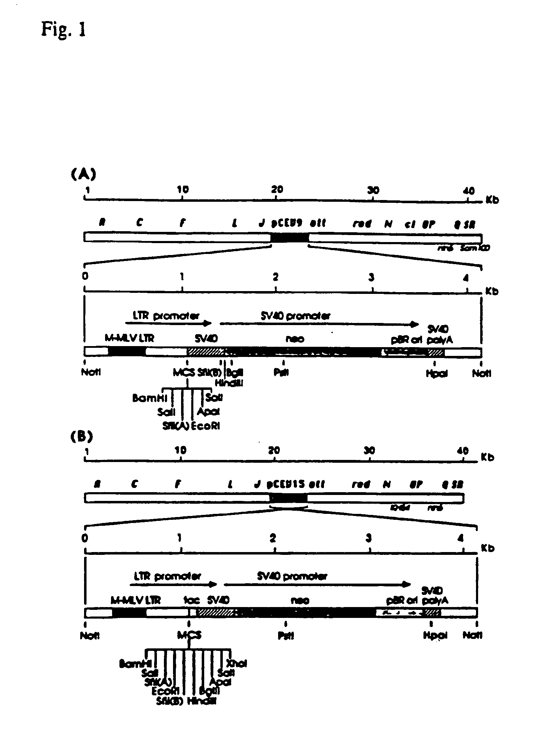

Still further, the present invention relates to a vector having nonidentical non-symmetrical restriction enzyme recognition site sequences, as described above, also including regulatory elements located such that the sequences of an inserted DNA segment are transcribed, as in the LambdaGEM™ vectors above, but where the regulatory elements are at least partly of eukaryotic origin. A principal embodiment of this aspect of the present invention is exemplified by two λ-plasmid composite vectors, λpCEV15 and λpCEV9, the structures of which are depicted in Figure 1 and described further below.

In cloning operations with these two vectors, a DNA segment, a cDNA, for example, is cloned between two *Sfi*I sites, A and B, as described in the section below relating to the automatic directional cloning method. The vectors are designed as eukaryotic expression vectors, utilizing the M-MLV LTR promoter, and they contain the SV40 early promoter-driven *neo* gene as a selectable marker.

Thus, the present invention further relates to a genetic cloning vector comprising at least one replicon, and a site for inserting DNA segments to be cloned that includes at least two nonidentical restriction enzyme recognition sequences that are non-symmetrical, where the vector also includes a selectable marker that is functional in eukaryotic cells in which the vector can be replicated. The term "functional" as used here means that the gene for the marker is expressed and that a selection scheme for that marker is operable in these eukaryotic cells.

In these two particular exemplary vectors, the form of λ-plasmid composite vector was chosen to take advantage of the efficient packaging and high density screening in λ systems, and simpler DNA preparation and analysis in plasmid systems. After isolation of clones of interest, pCEV plasmids with cDNA inserts can be obtained by *Not*I digestion of crude λ DNA preparations and ligation followed by transformation of bacterial cells. The λ genotype which supports healthy growth (*red*⁺, *gam*⁺) was chosen to maintain the intactness of inserts during the amplification step of the libraries. Deletion and/or insertion derivatives generated during the amplification step, if any, would not accumulate in the population, since they would not have a growth advantage.

λpCEV15 has several advantages over λpCEV9 as follows: (1) λpCEV15 does not require the *supF* mutation in host cell due to the *S*⁺ allele in the λ genome. (2) λpCEV15 does not lysogenize host strains due to the deletion of the *cI* gene. (3) cDNA inserts in λpCEV15 can be cut out by *Sal*I digestion. (4) λpCEV9 loses the functional SV40 promoter after the cDNA insert is cloned, while λpCEV15 does not. (5) λpCEV15 can accommodate longer cDNA inserts (up to 10.5 kb) than λpCEV9 (up to 8.5 kb). (6) λpCEV15 DNA contains a unique *Hind*III site.

λpCEV9 has at least two advantages over λpCEV15. The λpCEV9 genome has a stuffer fragment between the two *Sfi*I sites, which would be replaced by cDNA inserts during cloning process. Generally, it has been found that λpCEV9 cDNA libraries have lower backgrounds than λpCEV15 libraries, presumably because the presence of the stuffer fragment in λpCEV9 separates the two *Sfi*I sites enough to ensure complete *Sfi*I cleavage. It has also been observed that λpCEV9 grows more stably without accumulation of fast-growing derivatives. This is likely due to the longer size of the genome compared to that of λpCEV15.

In another aspect, the present invention relates to a method for cloning of DNA segments referred to as the automatic directional cloning method. In particular, the present invention relates to a method for cloning a cDNA copy of a eukaryotic mRNA, comprising the following steps (which are further illustrated in Figure 3 and in the Description of Specific Embodiments, below):
(i) annealing a linker-primer DNA segment comprising a single-stranded oligonucleotide which has oligo(dT) at the 3' end, and a single-stranded extension at the 5' end that is included in a first non-symmetrical restriction enzyme recognition sequence. [Note that this first recognition sequence is identical to one of two non-symmetrical sites in the vector that are used for direction cDNA cloning.]
(ii) enzymatically synthesizing the first strand of the cDNA from the linker-primer that is annealed with the mRNA molecule; [Typically, this may be accomplished using a reverse transcriptase. During the first strand synthesis reactions, the single-stranded linker-primer is repaired so as to be double-stranded. Thus, the single-stranded extension referred to in this method may be present as such in the linker-primer, or it may be produced from a double-stranded region of linker-primer by cleavage with a restriction enzyme following ligation of the linker-primer to the cDNA.]
(iii) enzymatically synthesizing the second strand of the cDNA using the first strand as the template under conditions such that single-stranded extensions on the synthesized cDNA molecule are made double-stranded; [Typically, the second strand is synthesized by DNA polymerase I from the nicks on the RNA moiety introduced by RNase H associated with the reverse transcriptase. The linker-primer is converted to the double-stranded form in the first or second strand synthesis step. T4 DNA polymerase treatment makes double-stranded any single-stranded extensions remaining on the synthesized cDNA molecule.]
(iv) ligating onto the blunt-ended cDNA resulting from synthesizing the second strand, an adaptor DNA segment comprising a second non-symmetrical restriction enzyme recognition sequence that is nonidentical to the first non-symmetrical restriction enzyme recognition sequence; [In the case of a principal embodiment of this aspect of the invention, ligation of the adaptor ligation directly adds one single-stranded extension to the cDNA molecule. Alternatively, however, this extension could be exposed by cleavage of the recognition site on a double-stranded portion of the adaptor after ligation to the cDNA.]
(v) exposing the cDNA resulting from ligation with the adaptor to one or more restriction enzymes that can cleave the first and second non-symmetrical restriction enzyme recognition sequences under conditions such that both of these sequences are cleaved, resulting in the vector DNA having two single-stranded ends that are not complementary;
   [This restriction causes exposure of at least one of the single-stranded extensions needed on the cDNA by cleavage of the recognition site on the repaired linker-primer portion of the cDNA molecule. If the non-symmetrical site in the adaptor is also uncleaved at this point, it may also be restricted at this step. In a principal embodiment, a single enzyme can cleave the non-symmetrical sites on both the linker-primer and the adaptor; but in other embodiments, two different enzymes may be required.]
(vi) ligating the cDNA resulting from cleavage with the enzymes to DNA of a genetic cloning vector, where the vector comprises
   at least one replicon; and
   a site for inserting DNA segments to be cloned that includes at least two non-symmetrical restriction enzyme recognition sequences,
   and where in the vector DNA, at least two non-symmetrical restriction enzyme recognition sequences have been cleaved by one or more enzymes that can cleave those recognition sequences, resulting in vector DNA having two single-stranded ends that are not complementary; wherein further,
      [Thus, the two ends of the cleaved vector DNA cannot anneal and be ligated together. Cleavage of the vector DNA at both non-symmetrical sites usually releases a short DNA segment from between the, the "stuffer"; for the highest yield of clones containing cDNA inserts, this stuffer is removed from the cleaved vector DNA prior to ligation of the vector with cDNA.]
   one of the single-stranded ends on the cleaved vector DNA has a sequence that is complementary to the single-stranded extension on the linker-primer attached to the cDNA; and
   the other single-stranded end on the cleaved vector DNA has a sequence that is complementary to the single-stranded extension on the adaptor attached to the cDNA; and
      [Thus the cDNA cannot circularize and is attached to the vector in a specific direction.]
(vii) transforming a suitable host cell with the recombinant DNA segment comprising the cDNA and the vector DNA that results from the ligation of cDNA to vector DNA; and
   [Various genetic transformation methods known in the art may be used. In a principal embodiment, the vector is a form of bacteriophage λ and, therefore, the recombinant DNA containing cDNA inserts is packaged *in vitro* into phage particles which are then used to infect a bacterial host cell. Alternatively, for example, CaCl₂ precipitation of DNA may be used to transform host cells, especially mammalian cells.]
(viii) identifying a clone of host cells, resulting from transformation with said recombinant DNA, that contains a recombinant DNA segment including said cDNA.
   [Various strategies well known in the art of genetic engineering may be used to identify a clone of the desired cDNA, including hybridization with nucleic acid probes, immunological detection of expressed antigens, and assays for functional products, to name but a few.]

The strategy underlying this cDNA cloning method of the present invention is based on the following theory, explained in terms of particular examples of a principal embodiment, which is presented to aid in understanding the method and does not in any way limit the scope of the invention as defined by the appended claims.

When vector and insert DNA fragments are mixed and ligated in a typical cloning experiment, several molecules are produced in addition to those desired. These include self-ligation products of vectors or inserts, head-to-tail or head-to-head dimers of vector or insert, and vector DNA containing multiple inserts. Formation of these molecules would reduce the cloning efficiency. Even when vector and insert DNAs are made by cutting with two different enzymes, formation of ligation products such as head-to-head dimers can not be eliminated, although the population of desired molecules is significantly higher and insertion occurs in a defined orientation.

The reason why these self-ligation products and dimers are made, as noted above, is that majority of restriction enzymes in common usage recognize sequences of dyad symmetry. The two sticky ends (S⁺ and S⁻) created with an enzyme contain the same single-stranded extensions, and all combinations of the ends including S⁺ and S⁺ can be ligated. However, certain restriction enzymes cleave the non-symmetrical site (A), yielding two different sticky ends (A⁺ and A⁻). In this case, only A⁺ and A⁻ ends can be ligated (see Fig. 2). When a vector DNA containing two different sites (A and B) with this feature is cleaved by restriction enzymes of this kind, the stuffer fragment hemmed by the sites is removed, and ligation is performed with inserts having sticky ends complementary to those of the vector, theoretically all of the clones obtained contain single inserts in the defined orientation.

In a principal embodiment of this aspect of the invention, the restriction enzyme *Sfi*I was chosen to cleave both the A and B sites, because *Sfi*I is an infrequent cutter and leaves a non-symmetrical 3' extension of three nucleotides (Fig. 2A). Since the central 5 bases in the recognition site can be any sequence, two *Sfi*I sites (A and B) were designed and introduced into the vectors (Figs. 1 and 2B). The cDNA fragments to be inserted into the vectors were oriented by the use of oligo(dT) primers having attached the sequence of the *Sfi*I(B) site.

The steps for cDNA synthesis are schematically shown in Fig. 3. During the first strand synthesis reactions, the single-stranded linker-primer is repaired so as to be double-stranded. After cDNA molecules are blunt-ended, an adaptor, designated *Sfi*I adaptor, having the 3' extension which fits to the *Sfi*I (A⁺) end, is ligated. After cleavage by *Sfi*I, the resulting cDNA molecules have different 3' extensions which fit on the vector ends to achieve directional cloning (Fig. 2C).

Thus, regardless of the sequences of the three-base single-stranded sticky ends, inversion of one *Sfi*I end sequence can never produce a self-complementary sequence. Accordingly, regardless of the sequence of the five arbitrary internal base pairs within any *Sfi*I cleavage site, the polarity of the complementarity of the sticky ends will always be maintained. Thus, such inherently non-symmetrical sticky ends, as well as non-symmetrical variants of recognition sequences for which some forms can have dyad symmetry, as described above (e.g., *Bst*XI), are also useful for practicing the automatic directional cloning method of the present invention, for efficient ligation of DNA fragments in a predirected order. Screenings of cDNA libraries constructed by this method, as described below, demonstrated that cDNAs of up to 6.4 kilobase pairs containing complete coding sequences could be isolated at high efficiency. Thus, this cloning system is particularly useful for the isolation of cDNAs of relatively long transcripts present even at low abundance in cells.

The present invention further relates to DNA of a genetic cloning vector comprising at least one replicon; and a site for inserting DNA segments to be cloned that includes at least two nonidentical restriction enzyme recognition sequences that are non-symmetrical, in which the non-symmetrical restriction enzyme recognition sequences have been cleaved by one or more enzymes that can cleave them, so that the DNA is ready for use in cloning DNA segments having matching sticky ends.

In a specific embodiment, exemplified below, the present invention relates to a cloning vector suitable for use in cloning DNA segments for cDNAs, by means of stable phenotypic changes induced by a specific DNA segments. An example of such vectors is λpCEV27 which differs (described above) as follows:
(1) The M-MLV LTR fragment is replaced by one derived from pZIPneoSV(X); one skilled in the art will appreciate, however, that similar fragments can be used.
(2) The bona fide promoter of the neo gene is removed to fuse the SV40 promoter directly to the neo structural gene. This modification eliminates ATG codens upstream from the translation initiation site of the neo gene, thereby increasing expression of the neo gene in mammalian cells. One skilled in the art will appreciate that the neo gene can still be expressed from the trp-lac fused promoter in bacteria (i.e., E. coli).
(3) The second selectable marker in bacterial cells, the ampicillin resistance gene (amp), is introduced to permit select transformed bacterial (i.e., E. coli) cells resistant to both ampicillin and kanamycin, thus avoiding selection of truncated plasmid clones. One skilled in the art will appreciate that alternative markers can be used.
(4) The sites for two additional infrequent cutters, Xhol and Mlul, were included along with the Notl site. Alternative infrequent cutters can also be used to effect the purpose of efficiently effecting plasmid rescue.
(5) The multiple cloning site (MCS) contains the restriction sites for BamHI, Sall, Sfil(A), EcoRI, Bgfl, Hindlll, Sfi(B), Sall, and BstEll, in more convenient order.
(6) The SP6-P and T7-P phage promoters were introduced to synthesize sense and anti-sense RNa of cDNA inserts, respectively. A Alternative promoters can also be used.
(7) A phage origin was introduced to synthesize single-stranded DNA from the vector, in the case of λCEV27, fl is used.
(8) The rat preproinsulin gene polyadenylation signal is added for efficient expression of DNA inserts (alternative signals can be used to effect the desired end result).
(9) The replication origin of pUC19 is used to increase the copy number of pCEV27. The replication origin of pCEV9 and pCEV15 is derived from a short fragment of pBR322; since the ori sequence lacks the promoter for replication initiation, unstable replication results and thus lower copy number. Replication origins similar to pUC19 can also be used to increase copy number.

Finally, the present invention also relates to a reagent kit comprising cleaved vector DNA, ready for use in cloning, as described above, and further including a linker-primer having a single-stranded end that is complementary to one single-stranded end of the cleaved vector DNA; and an adaptor which after cleavage by a suitable restriction enzyme, has a single-stranded end that is complementary to the other single-stranded end of the cleaved vector DNA. One skilled in the art of genetic engineering would appreciate that such a kit might advantageously also include appropriate quantities of enzymes, buffers and other reagents needed for the practice of the automatic directional cloning method according to the teachings of the present invention.

The present invention may be understood more readily by reference to the following detailed description of specific embodiments and the Examples and Figures included therein.

### BRIEF DESCRIPTION OF THE DRAWINGS

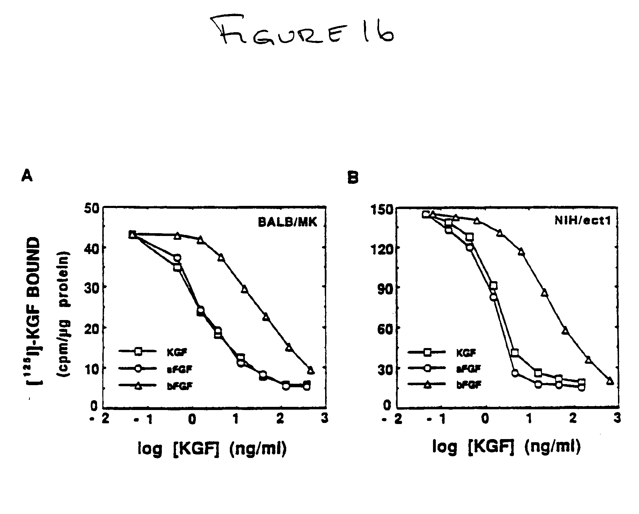

Fig. 1. Structures of the vectors. Panel (A), λpCEV9 and panel (B), λpCEV15. Each vector contains a plasmid DNA within the λ DNA. An expanded map of the plasmid portion is shown with derivation of the DNA segments and the location of several restriction sites including the multiple cloning site (MCS). Arrows show the locations of the promoters and the direction of transcription.
Fig. 2. Scheme of the automatic directional cloning system. Panel (A), Nucleotide sequences of the *Sfi*I sites. The general structure of the *Sfi*I site is shown at the top, where the letter N denotes any nucleotide. The two *Sfi*I sites specific to the vectors, *Sfi*I(A) and *Sfi*I(B), are shown under the general structure. The upper strands are shown in the 5' to 3' direction, while the lower ones are in the opposite direction. The bottom of the figure shows the sequences of the ends produced by the *Sfi*I cleavage of the general *Sfi*I site. The left and right half sites are denoted as *Sfi*I(⁺) and *Sfi*I(⁻), respectively. Similarly, the sequences of *Sfi*I(A⁺), *Sfi*I(A⁻), *Sfi*I(B⁺), and *Sfi*I(B⁻) half sites can be derived from the sequences of the *Sfi*I(A) and *Sfi*I(B) sites. Panel (B), preparation of the λpCEV vector arms. λpCEV vector DNA is shown at the top, where *cosL* and *cosR* represent the left and right cohesive ends of λ, respectively. The locations of the *Sfi*I(A) and *Sfi*I(B) sites are shown as (A) and (B), respectively. Following ligation to seal the cohesive ends (in the middle), the DNA is cleaved by *Sfi*I to expose the *Sfi*I(A⁺) and *Sfi*I(B⁻) sticky ends of the vector molecules. The small stuffer fragment is removed to prepare the vector arms shown at the bottom. The sequences of the single-stranded extensions are shown at both the 3'-ends of the cDNA and vector arms. Panel (C), formation of the λ concatemers containing cDNA inserts. The cDNA fragment shown at the left side are prepared to give the *Sfi*I(A⁻) and *Sfi*I(B⁺) sticky ends to the molecule by the procedure described in Fig. 3. When the fragments are ligated with the prepared vector arms, alternating concatemers consisting of the cDNA inserts and vector arms in the defined orientation are produced automatically due to the base-pairing specificity as shown in the middle. *In vitro* packaging extracts cut out the DNA segments hemmed by the two *cos* sites from the concatemer to form the active λ phage particles as shown at the bottom.
Fig. 3. Schematic view of the cDNA synthesis. An mRNA molecule is shown at the top with the cap structure (m⁷Gppp) and the poly(A) stretch (AAAAA) at the 5'- and 3'- ends, respectively. The linker-primer is the single-stranded oligonucleotide which contains the oligo(dT) at the 3' half, and the *Sfi*I(B) site (shown by an asterisk) at the 5' half. The first strand is synthesized by Moloney murine leukemia virus reverse transcriptase (M-MLV RT) from the linker-primer hybridized with the RNA molecule. The second strand is synthesized by DNA polymerase I from the nicks on the RNA moiety introduced by RNase H. The linker-primer is converted to the double-stranded form in the first or second strand synthesis step. T4 DNA polymerase treatment makes double-stranded any single-stranded extensions remaining on the synthesized cDNA molecule. The *Sfi*I adaptor ligation adds one 3' single-stranded extension, the *Sfi*I(A⁻) sticky end, to the cDNA molecule. Another 3' extension, the *Sfi*I(B⁺) sticky end, is exposed by *Sfi*I cleavage of the *Sfi*I(B) site on the repaired linker-primer portion of the cDNA molecule.
Fig. 4. Cloning of a model insert into pCEV15 using the ADC method. Panel (A), restriction map of pCEV15-*RAS*. The plasmid was constructed by cloning a 0.7 kbp fragment containing the mouse *H*-*ras* (*v*-*bas*) coding sequence (Reddy et al., 1985) into the *Eco*RI site of pCEV15. The open thick arc and closed thin arc represent the *H*-*ras* insert and vector, respectively. Panel (B), analysis of ligation products. pCEV15-*RAS* DNA was digested with *Sfi*I, and vector (4.2 kb) as well as insert (0.7 kb) fragments were purified from the gel. Similarly, *Eco*RI/*Apa*I fragments were prepared as controls. The vector and/or *H*-*ras* insert *Sfi*I fragments (left half) or *Eco*RI/*Apa*I fragments (right half) were incubated in kinase ligase buffer (see below) with or without T4 DNA ligase as indicated. The ligation products were analyzed by agarose gel electrophoresis. Sizes of the fragments are shown in kb. Panel (C), *Hind*III digestion of pCEV15 and its derivatives. (lane a) pCEV15; (lane b) pCEV15-RAS ; (lane c) pCEV15 containing the *H*-*ras* insert in opposite orientation; (lane d) a marker (1-kb ladder; Bethesda Research Laboratories); and (other lanes) Plasmid DNAs isolated from 20 individual kanamycin-resistant colonies obtained by transformation of DH5a with the vector ligated to *H*-*ras* insert *Sfi*I fragments.

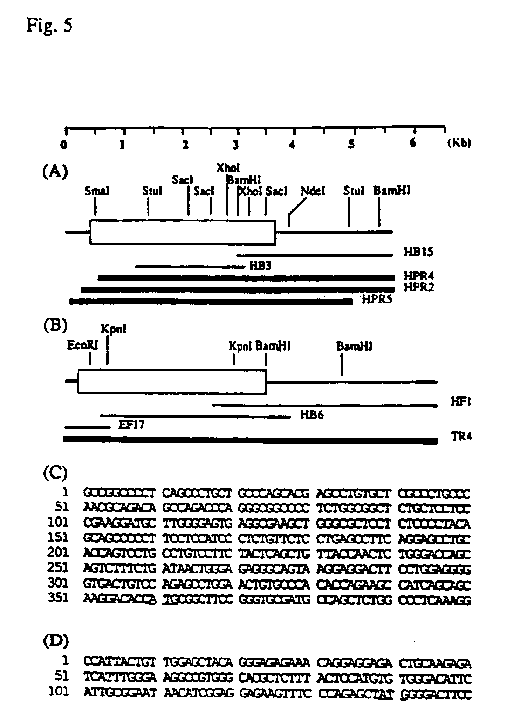

Fig. 5. PDGF receptor clones isolated from the λpCEV9-M426 cDNA library. Panels (A) and (B), cDNA clones encoding for β and α PDGF receptors, respectively. The structure of each PDGF receptor cDNA is schematically shown with restriction sites. Open boxes represent coding sequences, while non-coding sequences are shown by bars. The clones shown by thick lines were isolated from the M426 cDNA library. The thin lines represent clones isolated from other libraries as described (Matsui,T., et al., 1989, Science *243*, 800-804): HB15, HB3, and HB6 were derived from the human brain stem cell cDNA library in λgt11 (provided by R. Lazzarini; Matsui et al., 1989, *supra*); HF1 from the Okayama-Berg human fibroblast cDNA library (Okayama and Berg, 1982); and EF17 from a randomly-primed M426 cDNA library in λgt11 (Matsui et al., 1989, *supra*). Panels (C) and (D), nucleotide sequences of 5'-untranslated regions of β and α PDGF receptor clones HPR5 and TR4, respectively. Sequencing was performed by the chain termination method (Sanger et al., 1977, Proc. Natl. Acad. Sci. USA *74*, 5463-5467). The initiation codons are underlined.
**Figure 6. Structure of the cDNA cloning-expression vector λpCEV27.**
   Structure of the λpCEV27 genome is shown at the upper half with the location of l genes. The plasmid part is enlarged and shown at the lower half as a circular map. The multiple excision site (MES) contains the restriction sites for infrequent cutters; NotI, XhoI, PvuI, and MluI. The multiple cloning site (MCS) contains the restriction sites for BamHI, SalI, SfiI(A), EcoRI, BglII, HindIII, SfiI(B), SalI, and BstEII, and was placed in the clockwise orientation. The two SfiI sites are used to insert cDNA molecules by the automatic directional cloning method (Miki, T., et al. (1989) *Gene* 83, 137-146.), and the two SalI sites are used to release the inserts. SP6-P and T7-P represent the phage promoters for SP6 and T7 RNA polymerases, respectively. The trp-lac fused promoter tac and SV40 early promoter are used to express the neo structural gene in E. coli (kanamycin resistance) and eukaryotic cells (G-418 resistance), respectively. The directions of transcription from the promoters are shown by the arrows. Polyadenylation signals are labeled as polyA. The locations of the replication origins (ori) and the ampicillin resistant gene (amp) are shown.

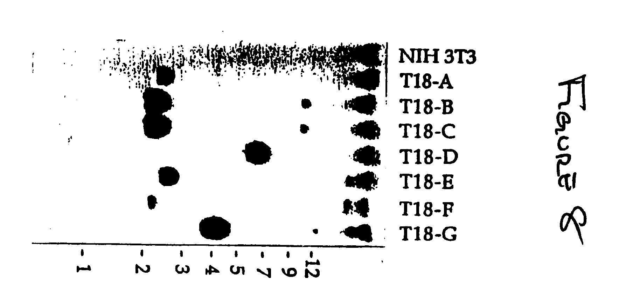

**Figure 7. Strategies for expression cloning of transforming gene cDNAs.**
   Cells of NIH 3T3 are transfected by λpCEV27 cDNA library DNA. Transformed cells are isolated from induced foci and assayed for G-418 resistance and colony formation in soft agar. Cells are expanded and the genomic DNA isolated. The DNA is digested by either NotI, XhoI or MluI, and then ligated in a low concentration. A bacterial strain is transformed by the ligated DNA and colonies resistant to both the ampicillin and kanamycin are isolated. Plasmid DNA is extracted from each colony and used to transfect NIH 3T3 cells to examine focus formation. Since cDNA library-induced foci is presumed to contain multiple cDNA clones, transforming plasmids are identified in this transfection assay.

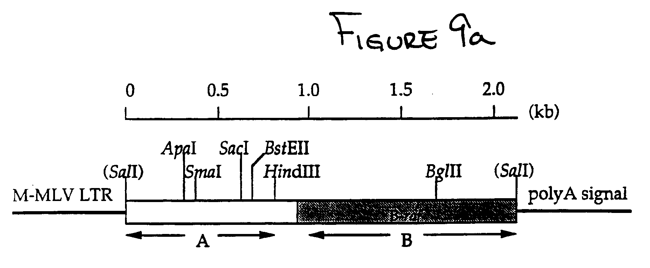

**Figure 8. Detection of hep1 cDNA inserts in the ST18-2 cDNA library-indcuced transformants.**
   The genomic DNA from individual transformants (from CT18-1A to CT18-1G) were digested by SalI which can release the cDNA inserts (see Figure 6). The digested DNA (5 mg) was separated on a 0.5% agarose gel by electrophoresis and transferred to a supported nitrocellulose membrane (Nitrocellulose GTG, FMC BioProducts). The Southern blot was probed by the hep1 cDNA insert of pHEP1-B which was rescued from the transformant T18-B. The NIH 3T3 genomic DNA was used as a negative control. The location of each fragment of the molecular size marker (1 kb ladder, BRL) is shown at the right side in kb.

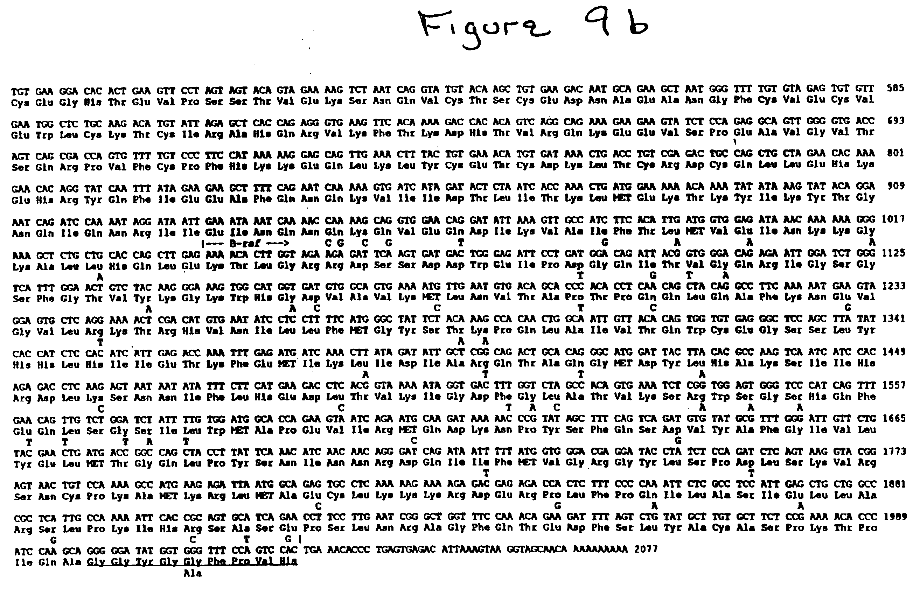

**Figure 9. Sequence homology of the hep1 and B-raf gene cDNAs.**
   A restriction map of the cDNA insert of pHEP1-B was schematically shown at the top(a). The regions where nucleotide sequence was determined are shown by arrows and labeled by A and B. The sequences are shown below(b). Computer analysis was performed by IntelliGenetics programs. B-raf sequence was taken from and numbered as in Ikawa et al. (1988).

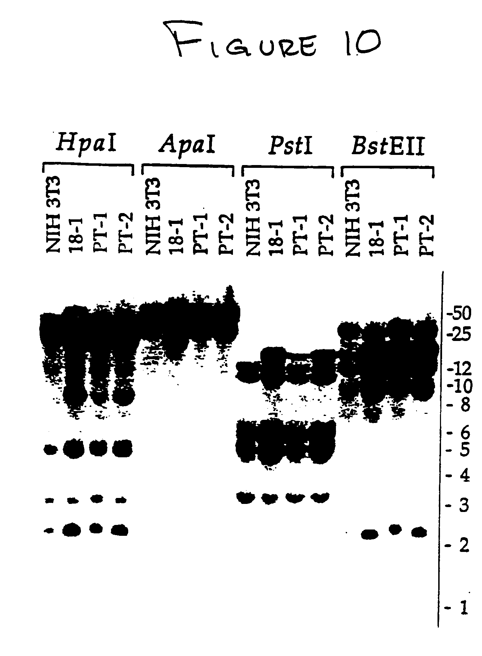

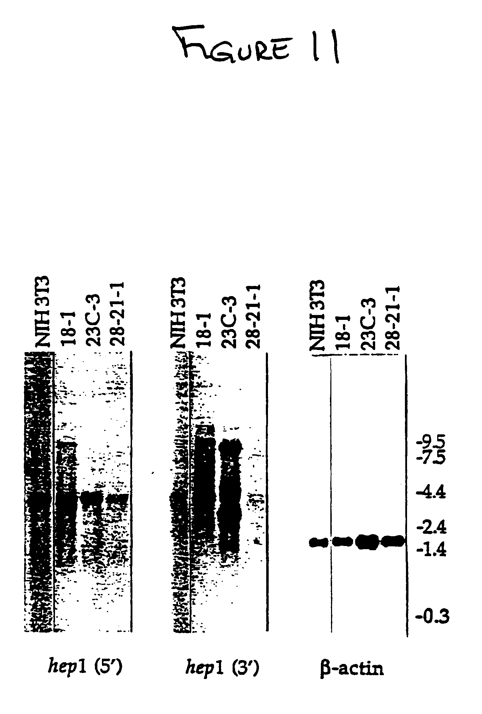

**Figure 10. Rearrangement and amplification of the hep1 locus in the primary and secondary transformants.**
   The sources of DNA and restriction enzymes used are shown at the top. The strains PT-1 and PT-2 are the primary transformants induced by the original tumor DNA. The strain 18-1 was a secondary transformant induced by PT-2 DNA and is the source of the cDNA library. The genomic DNA (5 mg) was digested by SalI to release the cDNA inserts, separated on a 0.5% agarose gel by electrophoresis, and transferred to a supported nitrocellulose membrane. The Southern blot was probed by the hep1 cDNA insert of pHEP1-B. The NIH 3T3 genomic DNA was used as a negative control. The location of several fragments of the molecular size markers (1 kb ladder and high molecular weight DNA marker, BRL) are shown at the right side in kb.

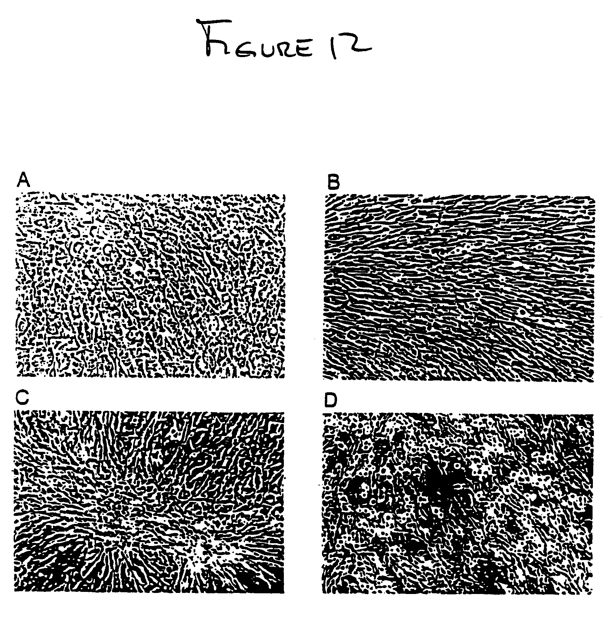

**Figure 11. Detection of mRNAs for the bra1 and B-raf genes.**
   The poly(A)⁺ RNA extracted from the cells indicated were denatured and separated on a formaldehyde gel. RNA was transferred to a supported nitrocellulose membrane and probed by each probe. The 5' probe was isolated as the SalI-HindIII fragment (see Figure 8). The 3' probe was prepared by polymerase chain reaction (PCR) using GeneAmp kit (Cetus Co.) from CT18-2B genomic DNA. The B-raf primer (5'-CCTCGAGATTCAAGTGATGAC-3') and the T7 primer (5'-CTAATACGACT CACTATAGGGG-3') were used for PCR and the amplified fragment was purified from an agarose gel.

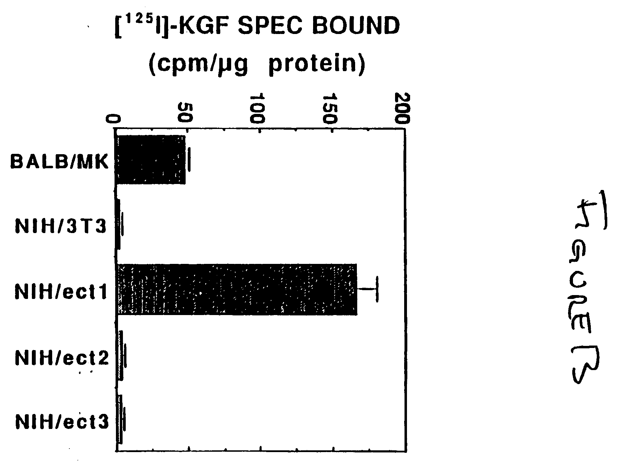

**FIG. 12 Cell morphology of control NIH/3T3 and transformants induced by keratinocyte cDNA expresion library.**
   NIH/3T3 cells (A) and NIH/3T3 cells transfected with the ect1 (B), ect2 (C), or ect3 (D) at 21 days post-transfection. Cells were maintained in Dulbecco's modified Eagle's medium (DMEM) containing 5% calf serum. (x 180)
**FIG. 13 Specific binding of [**^{**125**}**I]-KGF to BALB/MK, NIH/3T3, and NIH/ect1, NIH/ect2, and NIH/ect3 transformants.**
   **Methods:** Recombinant KGF was radiolabeled with [¹²⁵I]-Na by the chloramine-T method as described previously. Confluent cultures in 24-well plates were serum-starved for 24 h, followed by incubation with HEPES binding buffer (HBB; 100 mM HEPES, 150 mM NaCl, 5 mM KCl, 1.2 mM MgSO₄, 8.8 mM dextrose, 2 mg/ml heparin, and 0.1% BSA, pH 7.4) containing [¹²⁵I]-KGF for 1 h at 22°C. The cells were then washed with cold PBS, lysed with 0.5% SDS, and cell-associated radioactivity was measured in a gamma counter. Bound cpm were normalized to the cell protein content of SDS extracts. Specific binding was determined by subtracting normalized cpm of samples incubated with 100-fold excess unlabeled KGF from the normalized cpm bound in the presence of [¹²⁵I]-KGF alone.

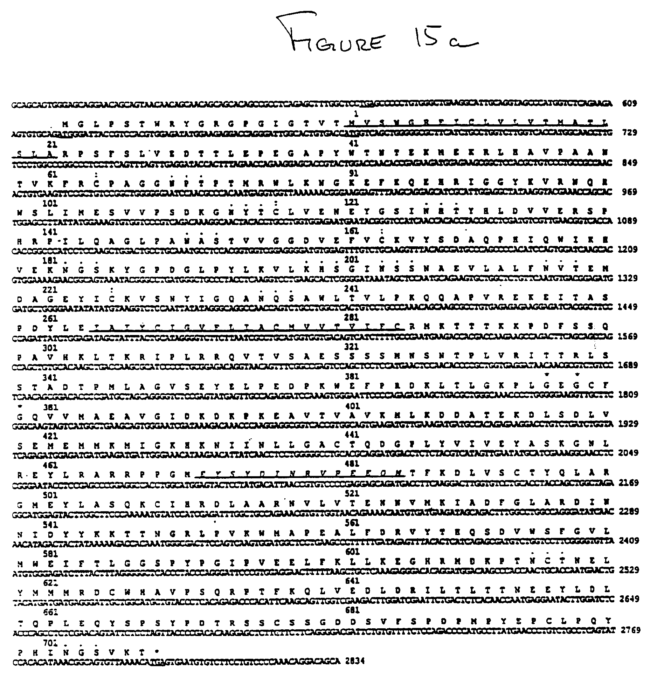

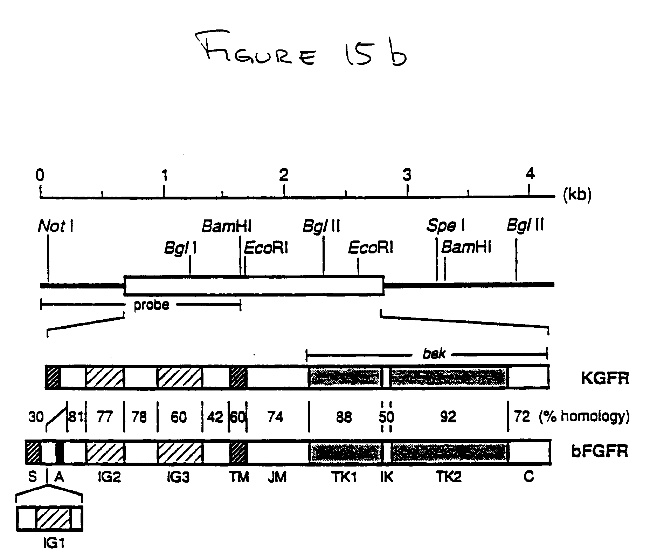

**FIG. 14 DNA and RNA analysis of the ect1 sequence. a,** Southern analysis of the SalI-digested DNAs from NIH/3T3 and its transformants. The blot was probed with the entire ect1 cDNA insert. Since SalI is an infrequent cutter of mammalian DNA, most of the DNA fragments are extremely large and migrate near the origin of the gel. However, the cDNA inserts released by SalI from the vector are shorter and migrate into the gel allowing the detection of the insert without detection of the endogenous ect1 gene.
   **b,** Southern analysis of EcoRI-digested DNAs of different animal species (Clontech, Palo Alto, CA). The blot was probed with the 5'-half of the ect1 cDNA insert and washed under reduced stringency conditions.
   **c,** Northern analysis of NIH/3T3 and BALB/MK RNA. The blot was probed with the 5'-half of the ect1 cDNA (lanes 1 and 2) or b-actin cDNA (lanes 3 and 4) and washed under stringent conditions.
      **Methods:** For plasmid rescue, genomic DNA was cleaved by one of the infrequent cutters which can release the plasmids containing cDNA inserts. Digested DNA was ligated under diluted conditions and used to transform bacterial competent cells. Plasmids were isolated from ampicillin- and kanamycin-resistant transformants and used to transfect NIH/3T3 cells to examine for focus formation. The ect1 plasmid was rescued by XhoI, while the ect2 and ect3 plasmids were rescued by NotI digestion. For Southern analysis, DNA (10 µg) was digested by SalI (Panel a) or EcoRI (Panel b), fractionated by agarose gel electrophoresis, and transferred to a nylon-supported nitrocellulose paper (Nitrocellulose-GTG, FMC, Rockland, ME). The blot in Panel a was hybridized with the ³²P-labeled entire ect1 insert at 42° C and washed at 65°C in 0.1 x SSC, while the blot in Panel b was hybridized with the ³²P-labeled 5'-ect1 probe (see Fig. 15b) at 37° C and washed at 55° C in 0.1 x SSC. Location of DNA molecular weight markers (BRL, Gaithersburg, MD) is indicated in kb. For Northern analysis (Panel c), poly(A)⁺ RNA (5 µg each) was fractionated by formaldehyde gel, transferred to Nitrocellulose GTG, and hybridized with the 5' ect1 probe (lanes 1 and 2). After autoradiography, the filter was boiled to remove the probe and then hybridized with a b-actin probe (Gunning et al Molec. Cell Biol. 3, 787-795 (1983)) (lanes 3 and 4). Location of molecular weight markers (BRL, Gaithersburg, MD) is indicated in kb.

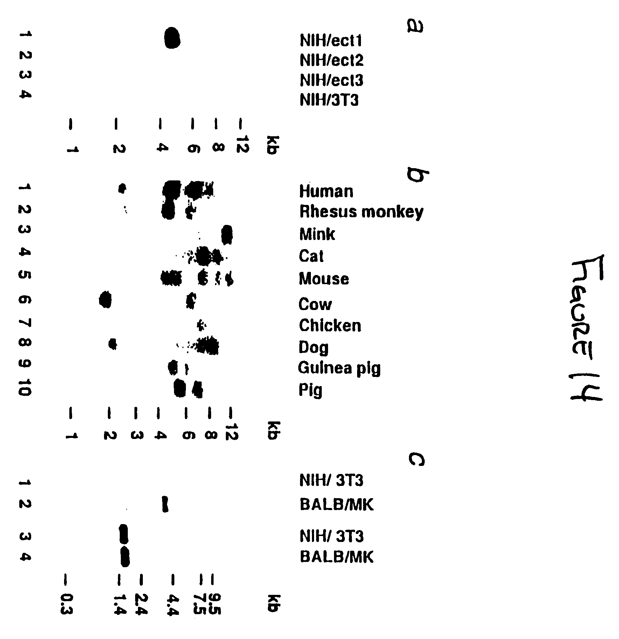

   All hybridization experiments were performed at the indicated temperature in a solution containing 50% formamide, 5 x SSC, 2.5 x Denhardt's solution, 7 mM Tris-HCl (pH 7.5), 0.1 mg/ml of denatured calf thymus DNA, and 0.1 mg/ml of tRNA.
**FIG. 15 Nucleotide sequence of the KGF receptor cDNA.**
   **a,** Nucleotide sequence and deduced amino acid sequence of the coding region of the KGF receptor cDNA. Nucleotides are numbered from the 5'-end of the cDNA. Initiation and termination codons are underlined. Amino acids are numbered from the putative initiation site of translation and shown above the amino acid sequence. Potential sites of N-linked glycosylation are indicated by dots above the residues. The potential signal peptide and trans-membrane domains are underlined. The interkinase domain is shown by underlined italic letters. Glycine residues considered to be involved in ATP binding are indicated by asterisks. Cysteine residues delimit two immunoglobulin-like domains in the extra-cellular portion of the molecule are shown by : over the residues. Nucleotide sequence was determined by the chain termination method (Sanger et al PNAS 74, 5463-5467 (1977)).
   **b,** Structural comparison of the predicted KGF and bFGF receptors. The region used as a probe for Southern and Northern analysis (Fig. 14b and c) is indicated. The region homologous to the published bek sequence^{3'} is also shown. The schematic structure of the KGF receptor is shown below the restriction map of the cDNA clone. Amino acid sequence similarities with the smaller and larger bFGF receptor variants are indicated. S, signal peptide; IG1, IG2, and IG3, immunoglobulin-like domains; A, acidic region;, TM, transmembrane domain; JM, juxtamembrane domain; TK1 and TK2, tyrosine kinase domains; IK, interkinase domain; C, C-terminus domain. Amino acid sequence comparison was performed using the method of Pearson and Lipman (Pearson et al, PNAS 85, 2444-2448 (1988)).

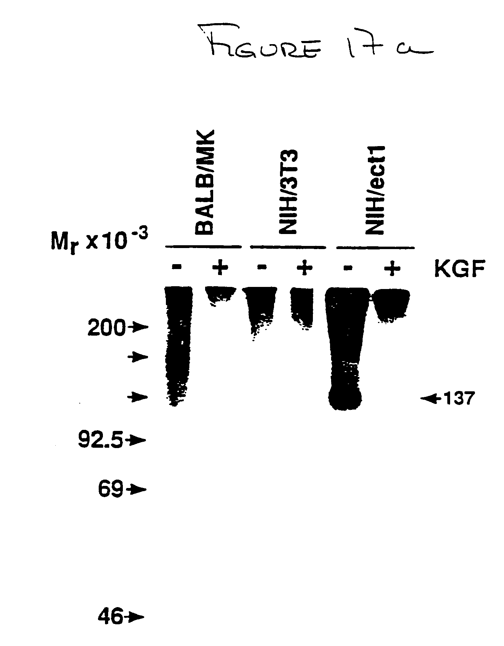

**FIG. 16 Competition of KGF, aFGF, and bFGF for [**^{**125**}**I]-KGF binding on BALB/MK cells (A) and NIH/ect1 cells (B).**
   **Methods:** Binding assays were performed as described for Fig. 14, except that cells were incubated with [¹²⁵I]-KGF in the presence of unlabeled KGF, aFGF or bFGF at concentrations indicated on the x-axis. For Scatchard analysis, samples contained several concentrations of [¹²⁵I]-KGF (1-100 ng/ml) in the presence or absence of a 100-fold excess unlabeled KGF, and were also processed as outlined in Fig. 13. Estimates of receptor affinity and total binding capacity were made using LIGAND software (Munson et al, Anal. Biochem. 107, 220-239 (1980)).

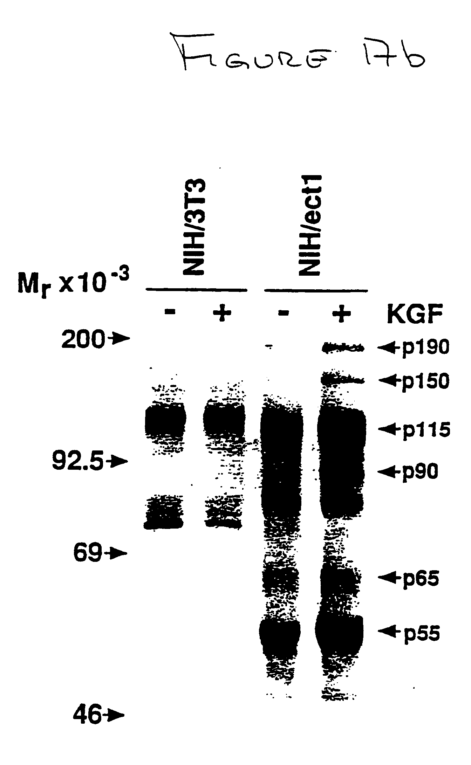

**FIG. 17 a,** Covalent affinity crosslinking of [¹²⁵I]-KGF to BALB/MK (left), NIH/3T3 (center), and NIH/ect1 cultures (right). The left and center panels of this autoradiogram were exposed to Kodak XAR film for 72 h at -70°C; the right panel is an 18 h exposure of the same autoradiogram. The second lane for each cell type shows crosslinking performed in the presence of excess unlabeled KGF. Molecular weight markers are indicated on the left; the positions of [¹²⁵I]-KGF-crosslinked complexes are indicated by arrows.
**b,** Autoradiogram of phosphotyrosyl-proteins from intact NIH/3T3 (left) and NIH/ect1 cells (right) before and after treatment with KGF. Molecular weight markers are indicated on the left; the estimated molecular weights of proteins displaying KGF-stimulated phosphorylation on tyrosine are shown at right.
   **Methods:** Samples for covalent crosslinking were prepared from confluent, serum-starved cultures in 6 cm dishes, using 10 ng/ml [¹²⁵I]-KGF in the presence or absence of 30-fold excess KGF. After binding (as described for Fig. 17), crosslinking with disuccinimidyl suberate was performed as described previously. The cells were then scraped into cold HBB containing 0.1mM aprotinin and 1.0 mM phenylmethylsulfonylfluoride, and a crude membrane fraction was generated by brief sonication (50 W, 10 sec), low-speed centrifugation (600 x g, 10 min), and high-speed centrifugation (100,000 x g, 30 min) of the low-speed supernatant. The membrane pellet was solubilized in Laemmli sample buffer (Laemmli, Nature 227, 680-685 (1970), containing 100 mM dithiothreitol, and boiled for 3 min. [¹²⁵I]-labeled proteins were resolved by 7.5% SDS-PAGE and autoradiography of the dried gel. Analysis of phosphoproteins was performed as follows. Confluent cultures in 10 cm dishes were serum-starved for 24 h, then treated with (+) or without (-) KGF (30 ng/ml) for 10 min at 37°C. The medium was aspirated, and the cells were solubilized in cold HEPES buffer containing 1% Triton X-100, protease- and phosphatase-inhibitors as described previously. The lysate was cleared by centrifugation, and phosphotyrosyl proteins were immunoprecipitated with affinity-purified anti-Ptyr adsorbed to GammaBind G-agarose (Genex, Gaithersburg, MD). Phosphotyrosyl proteins were specifically eluted using 50 mM phenyl phosphate, diluted in Laemmli sample buffer, and resolved by 7.5% SDS-PAGE. Proteins were then transferred to nitrocellulose and detected with anti-Ptyr and [¹²⁵I]-protein-A as described previously.

### DESCRIPTION OF SPECIFIC EMBODIMENTS

In one aspect, the present invention relates to a vector having nonidentical non-symmetrical restriction enzyme recognition site sequences, as described above, also including regulatory elements located such that the sequences of an inserted DNA segment are transcribed, where the regulatory elements are at least partly of eukaryotic origin. A principal embodiment of this aspect of the present invention is exemplified by two λ-plasmid composite vectors, λpCEV15 and λpCEV9, the structures of which are depicted in Figure 1 and described further below, in Example 1.

To examine the performance of the ADC method, a model H-*ras* insert was prepared so as to have *Sfi*I(A⁻) and *Sfi*I(B⁺) ends (Fig. 4A) and ligated with the pCEV15 *Sfi*I fragment. To show the difference between the ADC method and the "forced" cloning method using two different restriction enzymes, a similar H-*ras* fragment with *Eco*RI and *Apa*I ends was prepared (Fig. 4A) and ligated with the pCEV15 *Eco*RI/*Apa*I fragment. To measure the efficiency of cDNA cloning using a natural template, 2.5 mg of a poly(A)+ RNA preparation was denatured by heating and used to synthesize cDNA from a linker-primer. The results of all these experiments, described in Example 2, below, illustrate the remarkable efficiency of cloning of model inserts using this novel method of the present invention.

To assess the performance of the cDNA cloning method, cDNA was synthesized using poly(A)⁺ RNA extracted from M426 human embryonic lung fibroblast cells under the conditions described in Example 2, below. cDNA molecules larger than 1 kb were selected by low melting point agarose gel electrophoresis, and two aliquots were used to clone into λpCEV9 and λpCEV15. The average size of the cDNA inserts was 2.0 kb in the λCEV9 library (6 x 10⁶ independent clones) and 2.2 kb in the λpCEV15 library (1 x 10⁷ independent clones).

To characterize the M426 cDNA library in λpCEV9 further, it was screened for the human β PDGF receptor cDNA. Before this cDNA library was constructed, clones isolated (HB3 and HB15) by screening several other libraries did not contain the entire coding sequence (Fig. 5A). When a part (9 x 10⁵ pfu) of the M426 cDNA library was screened for the human β PDGF receptor, six clones were isolated. Of these, three contained inserts of approximately 5 kb. Sequence analysis showed that two (HPR2 and HPR5) contained the entire coding sequence (Fig. 5A). Recently, Matsui et al., 1989, *supra*) have identified the cDNA of a novel PDGF receptor, designated the α PDGF receptor by isolation of overlapping cDNA clones (HF1, HB6 and EF17 in Fig. 5B). Re-probing of filters from M426 cDNA library for the human a PDGF receptor resulted in the isolation of 93 clones. Of 7 clones analyzed, 5 including TR4 contained inserts of 6.4 kb, which corresponded to the size of the message (Fig. 5B). As shown in Figs. 5C and 5D, sequence analysis of α and β receptor cDNAs isolated from the M426 library revealed 5'- untranslated sequences followed by initiation codons for the complete coding sequence of each gene. These results indicated that the cDNA cloning system described here is suitable for isolation of relatively long cDNAs.

This method has been used for more than one year in the laboratory of these inventors, without public disclosure, to construct several cDNA libraries. Screening of the libraries for growth factors and receptors has been performed, and in most cases cDNA clones containing the entire coding sequence have been obtained as single clones. For example, a number of cDNA clones encoding keratinocyte growth factor have been isolated from the λpCEV9-M426 cDNA library using oligonucleotide probes. Screening of an MCF7 cDNA library in λpCEV9 constructed by the ADC method for a novel *erbB*-related gene resulted in the isolation of the cDNA clones of 5 kb with high frequency as well. All of these findings indicate that the ADC method using λpCEV vectors makes it possible to clone relatively long cDNAs very efficiently.

From the present data, the following conclusions may be drawn:
(1) The ADC procedure resulted in very high cloning efficiency ( 10⁷⁻10⁸ clones/µg of mRNA).
(2) Usually backgrounds of libraries constructed by this method are very low. When the vector arms are prepared carefully, almost all of the clones contain cDNA inserts.
(3) cDNAs are inserted into the vectors in a directed orientation and as single inserts, making analysis of cDNA inserts simple and straightforward.
(4) The vectors can accommodate longer inserts than other λ vectors without sacrificing cloning efficiency, making possible to clone relatively long cDNA fragments.
(5) Plasmids carrying cDNA inserts can be released from λ genomes by *Not*I cleavage. This feature facilitates the structural analysis of cDNA clones, permits the generation of size-selected plasmid sub-libraries, and makes it possible to recover the cDNA clones by plasmid rescue from eukaryotic cells.

The inventors have further refined the vectors to allow high levels of cDNA expression in mammalian cells and the ability to perform plasmid rescue. Example 3 tests the potential of the improved approach by its application to the isolation and characterization of unknown oncogenes from hepatocellular carcinomas of the B₆C₃F₁ mouse strain, extensively utilized in long term carcinogenesis testing in the United States.

Example 4 discloses the utility of the refined directional cDNA library expression vector for isolating the keratinocyte growth factor (KGF) receptor cDNA by creation of an autocrine transforming loop. This expression cloning approach was successful to identify and functionally clone the receptor for the new growth factor.

### Example 1. Construction of λpCEV15 and λpCEV9.

The following materials and methods were used in this and the subsequent examples, as needed.

Restriction enzymes, DNA polymerases, T4 DNA ligase, and T4 polynucleotide kinase were purchased from New England BioLabs, Bethesda Research Laboratories, and Boehringer Mannheim. M-MLV reverse transcriptase and RNaseH were from Bethesda Research Laboratories. Bacterial strain LE392; F⁻, *hsdR511(rk*^{*-*} *mk*^{*-*}*) supE44 supF58 lacY1 or D(lacIZY)6 galK2 galT22 metB1 trpR55* was used as a host of λ. DH5α (Bethesda Research Laboratories) was used for bacterial transformation. NZY broth (10g NZ amine, 5g NaCl, 5g Yeast extract in 1 l, pH 7.5) was used to grow bacterial strains. M426 is a human lung embryonic fibroblast cell line (Aaronson and Todaro, 1968, J. Virology *36*, 254-261). Oligonucleotides were synthesized by a Beckman System 1 DNA Synthesizer and purified by high performance liquid chromatography. Oligonucleotides utilized had the following sequences.
#1:GATCCGTCGACGGCCATTATGGCCAGAATTCTGGGCCCG,
#2:TCGACGGGCCCAGAATTCTGGCCATAATGGCCGTCGACG,
#3:AATTCAGGCCGCCTCGGCCAAGCTTAGATCTGGGCCCG,
#4:TCGACGGGCCCAGATCTAAGCTTGGCCGAGGCGGCCTG,
#5:TGGATGGATGG,
#6:CCATCCATCCATAA,
#7 and #8:GGACAGGCCGAGGCGGCC(T)n, where n=20 or 40 in the case of #7 and #8, respectively.

Plasmid DNA was prepared by the "selective precipitation procedure" which is a modification of the alkaline lysis method (Birnboim and Doly, 1979, Nucleic Acids Res. *7*, 1513-1523). This technique makes it possible to prepare sufficient pure plasmid DNAs to analyze and alter structures, without a requirement for lysozyme treatment, phenol extraction or repeated ethanol precipitations. Cells collected from a 10 ml culture were resuspended into 0.2 ml of TEG (25 mM Tris-HCl pH 8.0/10 mM EDTA/50 mM glucose). After transfer to a microcentrifuge tube, 0.2 ml each of 2% sodium dodecyl sulfate and 0.4 M NaOH was added, mixed, and incubated at room temperature for 5 min. After the addition of 0.2 ml of 3M ammonium acetate (pH 4.8), incubation at 0 C for 10 min, and centrifugation for 15 min in a microcentrifuge, the supernatant was transferred to a fresh tube containing 0.2 ml of 2 M Tris-HCl (pH 8.9) and 2 ml of 2 mg/ml RNase A. Following incubation at 37 C for 30 min and centrifugation, the supernatant was transferred to a new tube containing 0.6 ml cold isopropanol. The tube was inverted several times and incubated at room temperature for 10 min. DNA was collected by centrifugation and then washed with 75% ethanol. The pellet was dried by incubation at 37 C for 5 min and resuspended into 50 ml of 10 mM Tris-HCl (pH 8.0), 1 mM EDTA.

The λ DNAs prepared as follows were used to modify the structure, analyze cDNA clones in λpCEV vectors and then to rescue the plasmid part. Host cells grown in 10 ml of NZY medium containing 2 mM MgCl₂ were suspended into the same volume of SM buffer (50 mM Tris-HCl, pH 7.5/8 mM MgSO₄/100 mM NaCl/0.01 % gelatin). A single plaque picked by a pasteur pipet was incubated with 0.1 ml of the host cell suspension at 37 C for 30 min. Ten ml of pre-warmed NZY broth containing 2 mM MgCl₂ was added and shaken at 37 C for 6 h. This procedure allows single-step production of high-titer lysates. Phage particles were precipitated and DNAs prepared as described by Arber at al, 1983 [In Hendrix, R. W., Roberts, J. W., Stahl, F. W. and Weisberg, R. A. (Eds.), Lambda II. Cold Spring Harbor Laboratory, Cold Spring Harbor, NY,1983, pp. 433-466], with several modifications. A few drops of chloroform were added to the lysate, mixed and debris was removed by centrifugation. After the chloroform remaining in the lysate was removed by incubation at 37 C, 50 l each of DNase I (1 mg/ml) and RNase A (1 mg/ml) were added, and incubated at 37 C for 1 h. Phage particles were precipitated by the addition of 5 ml of 30% PEG/3 M NaCl/10 mM MgCl₂ followed by incubation on ice for 1 h. Phage particles were collected by centrifugation at 3,000 rpm for 30 min., and the pellet was resuspended in 0.5 ml of SM. The suspension was transferred to a fresh microcentrifuge tube containing 20 l of proteinase K (20 mg/ml). After incubation at 37 C for 15 min, 50 l of 100 mM Tris-HCl (pH8.0) / 100mM EDTA/ 1% sodium dodecylsulfate was added and the tube was incubated at 65 C for 30 min. The released DNA was extracted by phenol/chloroform and then by chloroform. DNA was precipitated by 0.6 volume of isopropanol with 0.3 volume of 7.5 M ammonium acetate, washed with 75% ethanol and dried. The pellet was dissolved in 10 mM Tris-HCl (pH 8.0), 1 mM EDTA, 0.1 mg/ml of RNase A and incubated at 37 C for 30 min to digest ribosomal RNAs.

Plasmid DNAs prepared by the selective precipitation method were directly used to modify the structures, by restriction enzyme digestions, repair reactions, and/or ligation with synthetic linkers. DNA fragments were separated on agarose gels and then purified using GENECLEAN (BIO 101 Inc.).

Insertion of oligonucleotides was performed as follows. The two strands of non-phosphorylated oligonucleotides were annealed and ligated with plasmid DNA which has been digested with suitable restriction enzymes. One strand of the oligonucleotides which was not ligated (due to the 5'-OH structure) was removed by heating and then separated by agarose gel electrophoresis. The purified fragments were phosphorylated and then ligated.

Plasmid pCEV9 was constructed as follows. A retroviral vector pDOL⁻ (Korman et al., 1987, Proc. Natl. Acad. Sci. USA *84*, 2150-2154) was cleaved by *Xba*I and recircularized to remove the polyoma segment. The *Cla*I site was converted to a *Not*I site by linker insertion, and the *Eco*RI site was removed by repair ligation. A synthetic MCS linker consisting of oligonucleotides #1 and #2 (see Example 1, below) was inserted between the *Sal*I and *Bam*HI sites, and the *Bcl*I/*Bam*HI fragment of SV40 DNA (Bethesda Research Laboratories) containing its polyadenylation signal was inserted at the *Xho*I site. These manipulations produced pCEV9.

λgt11 DNA (Young and Davis, 1983, Proc. Natl. Acad. Sci. USA *80*, 1194-1198) was ligated and then cleaved by *Eco*RI and *Xba*I. The ends were repaired and *Not*I-linkered, and then pCEV9 DNA linearized by *Not*I was cloned into the λ DNA to produce λpCEV9.

pCEV15 was constructed by modifying pCEV8, which is a parental plasmid of pCEV9 and lacks the MCS linker. A *tac* promoter fragment (Pharmacia) and *Xho*I linker were inserted in the *Sal*I site. *Sfi*I(B), *Hind*III, and *Bgl*II sites were removed successively by restriction enzyme digestion, polymerase treatment, and subsequent ligation reaction. Removal of the *Sfi*I site (on the SV40 replication origin) did not impair the SV40 early promoter activity. The MCS linker was inserted between the *Bam*HI and *Sal*I sites, and *Sfi*I(B), *Hind*III, and *Bgl*II sites were introduced again by insertion of the MCS-2 linker (oligonucleotides #3 and #4, below) between the *Eco*RI and *Apa*I sites. The resulting plasmid pCEV15 was cloned in a new λ vector constructed as follows. The segment spanning from the *Xho*I site to the right *cos* end of λpCEV9 was replaced by the corresponding fragment of λcharon28 (Rimm et al., 1980, Gene *12*, 301-309), to introduce the *cI* deletion (KH54) and remove three *Hind*III sites. The resulting phage λpCEV9c DNA was ligated, cleaved by *Hind*III, and then repaired. A *Not*I linker was ligated to the repaired *Hind*III ends and the DNA was cleaved by *Not*I. The λ arms were purified and ligated with *Not*I-digested pCEV15 DNA, to produce λpCEV15.

### Example 2. Efficiency of cloning and orientation of model inserts.

Preparation of λ arms and the *Sfi*I adaptor for all cloning experiments was performed as follows. λ DNA was ligated to seal cohesive ends and then cleaved sequentially by *Sfi*I and EcoRI. After phenol/chloroform extraction, λpCEV9 arms were purified by centrifugation through a 5-20% potassium acetate gradient (Maniatis et al., 1982). λpCEV15 arms were purified by passage through a Sephadex G-50 spin column (Boehringer Mannheim Biochem.).

The *Sfi*I adaptor was prepared as follows. About 1 nmol of oligonucleotides #5 and #6 were separately phosphorylated by T4 polynucleotide kinase, mixed, heated at 80°C for 5 min and then slowly cooled to 4°C.

RNA for making cDNAs was extracted and Poly (A)⁺ RNA selected as described by Okayama et al. (1987). cDNA was synthesized essentially according to D'Alessio et al. (1987) with some modifications. About 2.5 mg of poly(A)⁺ RNA in 10 ml of H₂O was mixed with 0.5 ml of 100mM methylmercuric hydroxide (MeHg), and incubated at room temperature for 5 min, followed by addition of 0.5 ml of 1.4M b-mercaptoethanol. After 5 min, 1.2 ml of RNasin (40 units/ml; Promega Biotech.), 17.8 ml of H₂O, 10 ml of 5x FS buffer (250mM Tris-HCl, pH 8.3/375mM KCl/15 mM MgCl₂/100 mM dithiothreitol), 2.5 ml of dNTP mixture (10 mM each of dGTP, dATP, dTTP and dCTP), 5 ml of linker-primer (oligonucleotide #8; 1 mg/ml) and 2.5 ml of M-MLV reverse transcriptase (200 units/ml) were sequentially added, mixed, and incubated at 37°C for 1 h. The tube was chilled on ice and 290 ml of H₂O, 7.5 ml of dNTP mix (10 mM each of dGTP, dATP, dCTP, and TTP), 40 ml of 10x SS buffer (188 mM Tris-HCl, pH8.3 / 906 mM KCl / 46 mM MgCl₂ / 38 mM DTT), 10 ml of DNA polymerase I (1.25 units/ml) and 1.8 ml of RNase H (0.25 units/ml) were added, mixed and incubated at 16°C for 2 h. The reaction mixture was heated at 70°C for 10 min, and 5 ml of T4 DNA polymerase (1 unit/ml) was added and incubated at 37°C for 10 min. The reaction was terminated by the addition of 40 ml of 0.25M EDTA, and the mixture was extracted by phenol/chloroform twice followed by chloroform twice. cDNA was ethanol-precipitated from 2.5 M ammonium acetate, washed, and then dried. The pellet was dissolved into 10 ml of H₂O and then 4 ml of *Sfi*I adaptor (0.8 mg/ml), 4 ml of 5x ligation buffer (500 mM Tris-HCl, pH7.6/100 mM MgCl₂ /10 mM ATP/10mM dithiothreitol/ 50% (w/v) polyethylene glycol-8000) and 2 ml of T4 DNA ligase (1 unit/ml) were mixed and incubated at 14°C overnight. A 10 ml aliquot was then mixed with 1 ml of 10x *Sfi*I buffer (100 mM Tris-HCl, pH 7.9 / 500 mM NaCl / 100 mM MgCl₂ / 60 mM b-mercaptoethanol / 1 mg/ml bovine serum albumin), 2 ml of *Sfi*I (10 units/ml) and 7 ml of H₂O. Digestion was performed at 50 C for 1 h. cDNA fragments were purified by low-melting point agarose gel electrophoresis or passing through a spun column (Maniatis et al., Molecular Cloning, Cold Spring Harbor 1982) packed with Sepharose CL-4B (Pharmacia). An aliquot of the cDNA preparation was then mixed with λpCEV vector arms and ethanol-precipitated. DNA was dissolved in 8 ml of H₂O, and then 1 ml of 10x kinase-ligase buffer (660 mM Tris-HCl, pH7.5/100 mM MgCl₂/50 mM dithiothreitol, 500 mM ATP) and 1 ml of T4 DNA ligase (1 unit/ml) were added, mixed, and incubated at 14°C overnight. In vitro packaging was performed using GigaPack Gold (StrataGene) as directed.

As shown in Fig. 4B, neither of the vector nor H-ras insert *Sfi*I fragment was self-ligated, while self-ligation occurred when the EcoRI/ApaI fragments were used instead. In the ADC system, ligation occurred only when both the vector and insert fragments were present in the reaction mixture (Fig. 4B). To characterize the directional cloning capacity of the system, the H-ras insert and vector *Sfi*I fragments were ligated, used to transform an E. coli strain DH5a, and 20 kanamycin-resistant colonies were analyzed. As shown in Fig. 4C, all plasmids contained single inserts in the expected orientation, indicating that the ADC method provides both directional cloning and positive selection for the presence of inserts. To further examine the performance of the ADC method using the λ system, model inserts prepared to have *Sfi*I(A⁻) and *Sfi*I(B⁺) ends were ligated with λpCEV9 arms (see Fig. 2C). As shown in Table I, pCEV9 arms alone did not produce active phages efficiently even when the ligation reaction was carried out, while presence of model inserts in

**Table I**

| Packaging efficiency of λpCEV9 DNA | |
|---|---|
| DNA | Titer^{a} (pfu/µg λ arms) |
| λpCEV9 arms | 1 x 10³ |
| λpCEV9 arms, ligated | 8 x 10³ |
| λpCEV9 arms + Insert A^{b}, ligated | 8 x 10⁶ |
| λpCEV9 arms + Insert B^{c}, ligated | 8 x 10⁶ |

| | |
|---|---|
| Footnotes for Table I: a The reaction mixture contained 66 mM Tris-HCl (pH7.5), 10 mM MgCl₂, 5 mM dithiothreitol, 50 mM ATP, and 0.1 µg/ml of DNA. Incubation was performed at 14°C overnight, and the phage were produced by *in vitro* packaging and titered on LE392. | |
| ^{b} A 2 kb DNA fragment having the *Sfi*I(A⁻) and *Sfi*I(B⁺) ends (see Fig. 2). | |
| ^{c} A DNA fragment similar to the insert A, except that the *Sfi*I(A⁻) end was created by ligation of the *Sfi*I adaptor. | |

the ligation mixture increased the titer of active phages by three orders of magnitude. These results indicated that successful ligation and phage propagation depended on the presence of the model insert in the reaction mixture. All of thse findings indicated that the cloning procedure results in low background and efficient directional cloning.

To measure the efficiency of cDNA cloning using a natural template, 2.5 mg of a poly(A)⁺ RNA preparation was denatured by heating and used to synthesize cDNA from a linker-primer (oligonucleotide #7). The cDNA was blunt-ended, and the *Sfi*I adaptor was ligated to both the ends. The molecules were cleaved partially by *Sfi*I, and then cloned in λpCEV9. A total of 2.5 x 10⁸ plaque forming units (pfu) was obtained, indicating that the method was extremely efficient.

Since *Sfi*I is an infrequent cutter, almost all cDNA species in the libraries constructed by the ADC method should remain intact. Nonetheless, cDNAs containing *Sfi*I sites might be excluded from our cDNA libraries. To solve the problem, partial *Sfi*I digestion is usually performed. An alternative strategy involves cDNA synthesis from a linker-primer containing the recognition site of another infrequent cutter *Mlu*I, in addition to the *Sfi*I site. The cDNA preparation could then be divided into two parts, one cleaved by *Sfi*I and the other by *Mlu*I. A short oligonucleotide ligated to the cDNAs could be utilized to convert the *Mlu*I end to an *Sfi*I(B⁺) end.

### Example 3. Isolation of a Mouse Hepatoma Oncogene cDNA Using a Novel Phenotypic Expression Cloning System.

The following protocols/methodologies are referred to in sections 3.1--3.6 below.

### cDNA Library Construction

cDNA libraries were constructed as described above except the use of newly-designed adaptor and vector. The new SfiI adaptor does not contain the ATG codon in the sense strand and was consisted of two oligonucleotides,
5'-CCAATCGCGACC-3' and 5'-GGTCGCGATTGGTAA-3'.
Amplification of the library and preparation of the DNA were performed by the standard procedures (Maniatis et al. 1982).

### Cell Culture and DNA transfection

All cells used were the derivatives of NIH 3T3 (Jainchill, J.L., et al. (1969) *J. Virol.* p. 549.). Calcium phosphate transfection (Wigler, M. et al., (1977) *Cell* 11, 223.) was used to introduce DNA into cells. Cells were maintained in Dulbecco's modified Eagle's medium (DMEM) containing 5% calf serum.

### Plasmid Rescue

The genomic DNA (1.2 µg) isolated from CT18-2B or CT18-2C was cleaved by XhoI, extracted with phenol-chloroform and then chloroform. The DNA was precipitated and resuspended in 355 µl of H₂O. Fifty µl of 10 x kinase-ligase buffer (660 mM Tris-HCl, pH7.5/100 mM MgCl₂/50 mM dithiothreitol, 500 µM ATP) and 5 units of T4 DNA ligase (BRL) were added and incubated overnight at 15 °C. The ligated DNA was extracted and precipitated as above and then resuspended in 10 µl of TE. Four aliquotes (100 µl each) of PLK-F' competent cells (Stratagene) were transformed by 0.5, 1, 2, and 4 µl of the ligated DNA as directed by the manufacturer. After the heat shock, the cells were dileted 10 fold with S.O.C. medium (BRL) containing 1 mM IPTG to induce expression of the neo gene drived by the tac promoter. The culture was incubated for 2 h with shaking and plated on NZY hard agar containing ampicillin (100 µg/ml), kanamycin (25 mg/ml), and IPTG (100 µM/ml).

### Recombinant DNA Techniques

Preparation of l and plasmid DNA was performed as described above. Genomic DNA was extracted by the standard procedure (Maniatis et al., 1982). Total RNA was isolated and poly(A)-selected as described by (Okayama et al. (1987) PNAS 84, 8573).

### Southern and Northern Analysis

DNA fragments were isolated by Geneclean (BIO101 Inc.) and labeled by random priming using Oligo Labeling kit (Pharmacia). Hybridizations were performed as described (Kraus et al., 1987).

### 3.1 Development of an Efficient Stable Expression cDNA Cloning System

The λpCEV27 system was developed to clone cDNAs by means of stable phenotypic changes induced by a specific cDNA. Use of a λ-plasmid composite vector made it possible to generate high complexity cDNA libraries and to efficiently excise the plasmid from the stably integrated phagemid DNA. This phagemid vector (Figure 6) contained several features including two SfiI sites for construction of cDNA libraries using the automatic directional cloning (ADC) method, an M-MLV LTR promoter suitable for cDNA expression in mammalian cells, the SV40 promoter-driven neo gene as a selectable marker, and multiple excision sites (MESs) for plasmid rescue from genomic DNA. The λ-pCEV27 system incorporated, in addition to the M-MLV LTR, the rat preproinsulin polyadenylation (polyA) signal downstream from the cDNA cloning site (Fig. 6). In this vector, the bacterial neo gene was placed under the independent control of the SV40 early promoter and the SV40 late polyA signal for use in marker selection in mammalian cells. In contrast to λpCEV15, the *bona fide* promoter of the *neo* gene was removed so as to fuse the SV40 promoter directly to the *neo* structural gene. Thus, in λpCEV15, expression of the neo gene in *E. coli* was achieved by transcription from the *trp-lac* fused promoter *tac,* inserted upstream from the SV40 early promoter (Fig. 6). By use of the tac promoter, it was possible to utilize IPTG-inducible selection for kanamycin resistance. Finally, the f1 replication origin and, SP6 and T7 phage promoters were included to facillitate analysis of cDNA inserts by production of single-stranded DNA and RNA transcripts, respectively (Fig. 6).

The strategy for expression cloning of oncogene cDNAs is summarized in Figure 7. When library cDNA is used to transfect mammalian cells, cDNA clones are integrated with recombination between l DNA and host genomic DNA. For plasmid rescue, genomic DNA extracted from transformant is subjected to digestion with an enzyme which can cleave the λ-plasmid junctions. The resulting DNA can then be circularized and used for bacterial transformation. For this purpose, the sites for two additional infrequent cutters, XhoI and MluI, were included along with the NotI site. Because of the second selectable marker in bacterial cells, the ampicillin resistance gene (amp), it was possible to select transformed E. coli cells resistant to both ampicillin and kanamycin, avoiding selection of truncated plasmid clones.

### 3.2 Characterization of Oncogenes Activated in Mouse Liver Tumors

We have previously analyzed hepatocellular tumors of the mouse strain B6C3F1 for the presence of activated oncogenes (Reynolds et al. (1987) Science 237, 1309-1316). Although the majority were activated ras or c-raf oncogenes, four could not be identified. The sources of these oncogenes were tumors designated OT4, OT18, OT23, and OT28. One (OT23) was spontaneously generated, while the others were associated with chronic furfural exposure (Reynolds, S.H., et al., (1987)). Genomic DNAs of NIH/3T3 transformants containing each of the unidentified oncogenes were examined under low stringency hybridization conditions using a number of known and potential oncogene probes including abl, myb, ets, fos, fgr, fms, rel, src, sis, yes, p53, ros, PDGF-A, met, dbl, lskT, myc, N-myc, rho, mos, erbA, pin, lca, H-ras, N-ras, K-ras, c-raf, and erbB-2. None showed DNA fragments with either increased intensity or abnormal sizes relative to those detected in NIH/3T3 control DNA (data not shown). Thus, none of these transforming genes appeared to be closely related to any of the genes used as probes.

### 3.3 Expression cDNA Cloning of an Oncogene of a Furfural-induced Hepatoma

Using the λpCEV27 expression cloning system, we attempted to clone transforming cDNA from one furfural-induced tumor, OT18. A cDNA library (3 x 10⁶ independent clones) was constructed from poly(A)⁺ RNA extracted from a secondary transformant of the tumor. Transfection of 80 plates of NIH/3T3 cells with 5 µg/plate of the expression cDNA library led to the detection of seven foci, which demonstrated G-418 resistance. These results indicated that each had taken up and stably integrated vector DNA, making it likely that the transformed foci were induced by exogenous cDNA rather than arising as a result of spontaneous transformation.

Two of these transformants, designated CT18-2B and CT18-2C, were selected for plasmid rescue. By restriction mapping of several distinct plasmids obtained from each transformant, it was possible to establish that one plasmid rescued from each had the identical insert (data not shown). These results suggested that this cDNA might encode the oncogene product. Transfection analysis of each rescued plasmid DNA demonstrated that these same two cDNA clones possessed high-titered transforming activity of around 10³ ffu/nmol DNA, while none of the other plasmids rescued from the same transformants showed detectable activity.

To determine whether other transformants induced by the cDNA library contained the OT18 oncogene, genomic DNA extracted from each primary transformant was digested with SalI to release cDNA inserts from the vector and subjected to Southern blotting analysis using the OT18 cDNA insert as a probe. Since SalI is an infrequent cutter for mammalian DNA, genomic DNA was cleaved to very large fragments which remained near the origin of the gel. Thus, the relatively small cDNA fragments released from cellular DNAs by SalI cleavage could be separated from the bulk of genomic fragments. As shown in Figure 8, each of the cDNA library transformants contained the OT18 oncogene cDNA insert. The sizes ranged from around 2.3 kb to 7 kb, suggesting that serveral of the inserts represented independent cDNA clones of the oncogene.

### 3.4 Structural Analysis of T18 oncogene cDNA

A detailed restriction map of the 2.1 kb insert of one of the transforming plasmids was constructed, and the clone was subjected to sequence analysis. A database search indicated that that the 5' portion of the cDNA contained an unknown sequence, while the 3' region was closely related to human B-raf (Ikawa et al., (1988) Mol. Cell. Biol. 8, 2651-2654), and chicken Rmil (Marx et al., (1988) EMBO 7, 3369-3373) genes (Figure 9a). Comparison of the predicted amino acid sequences with that of B-raf indicated identity (Figure 9) with the exception of a single amino acid difference at position 324, in which Gly was substituted for Ala in human B-raf.

There was also complete identity with avian R-*mil* except for a small stretch of nine amino acids at the R-*mil* C-terminus, where recombination with an avian retroviral *env* gene caused this substitution.

To determine the breakpoint, we also compared the T18 nucleotide sequence with that of proto B-*raf* and v-R-*mil*. There was no homology with either sequence upstream from position 1040 in the T18 oncogene. Thus, position 1040 represents the junction between an unknown sequence and the B-*raf* gene. R-*mil* is a viral onocogene and encodes a *gag*-R-*mil*-*env* fusion protein. The junction of *gag* and R-*mil* has been mapped 144 nucleotide upstream from the T-18 break point (Marx *et al*., 1988), while the junction of a different sequence and the human B-*raf* oncogene was 174 nucleotides upstream from the junction in the T18 oncogene. In each of the B-*raf* oncogenes, including T18, the breakpoints did not disrupt the predicted kinase domain of the protein.

### 3.5 Evidence for in Vivo Oncogene Activation

The human B-raf gene product is around 84% related to the amino acid sequence of the c-raf oncogene. Another member of the raf family, A-raf, is also structurally similar. Most raf oncogenes have been activated by mechanisms involving structural rearrangements due to recombination and loss of amino terminal sequences of the raf coding sequence (Rapp, U.R., et al., (1988) In: Reddy, E.P. (ed.) The Oncogene Handbook, Elsevier Science Publishers B.V.). Moreover, most reported instances have involved in vitro activation of these genes during DNA transfection rather than by mechanisms leading to oncogene activation within the tumor itself (Ishikawa, F., et. al., (1987) T. *Mol. Cell. Biol.,* 7, 1226); (Stanton, V.P. and Cooper, G.M. (1987) *Mol Cell*. *Biol*. 7, 1171); (Ikawa, S., et al., (1988) *Mol. Cell. Biol.* 8, 2651-2654). The rearrangement activating the B-raf oncogene might have occurred during the course of cDNA library construction or as an artifact of DNA transfection with the original tumor DNA. Alternatively, the oncogene might have been activated within the original tumor itself.

While original tumor DNA was not available, it was possible to analyze two primary transformants which had been independently induced by this tumor DNA. As shown in Figure 10, rearrangement as well as amplification of both non-B-raf and related B-raf portions of the gene were found not only in the secondary transfectant, which was the source of the cDNA library, but in both primary transformants, PT18-1 and PT18-2. Since such in vitro rearrangements are very rare, these findings strongly argue that the oncogene was activated in vivo in the hepatoma as part of the neoplastic process.

### 3.6 Detection of the mRNAs for the T18 Oncogene.

In an effort to characterize the B-raf oncogene transcript and search for evidence of additional B-raf oncogenes among the 3 other hepatoma oncogenes, we subjected polyA selected RNAs from primary or secondary NIH/3T3 transfectants containing each oncogene to analysis with DNA probe from 5' (B-raf unrelated) and 3' (B-raf) portions of the T18 oncogene. As shown in Fig. 11, control NIH/3T3 cells contained a 4.2 kb RNA that hybridized with the 5' non-B-raf related portion of the oncogene but there was no detectable B-raf transcript. In contrast, the second cycle T18 transfectant, which was the source of expression cDNA library, showed a major 4.2-kb as well as minor 10- and 3-kb transcripts which appeared to hybridize with both probes.

Fig. 11 further shows that a primary T23 oncogene transfectant contained multiple B-raf hybridizing transcripts, indicating that it also contained another B-raf oncogene. Of note, the several transcripts detected differed in their sizes from those of the T18 oncogene. Moreover, none of these transcripts was detected by the B-*raf* probe of the T18 oncogene (Fig. 11). Thus, if the T23 oncogene arose by a mechanism involving B-*raf* gene rearrangement, this rearrangement was different from that associated with activation of the T18 oncogene. The transfectant induced by the T28 oncogene (Fig. 11) did not show abnortmal B-*raf* hybridizing RNAs, arguing that this oncogene must be distinct from B-*raf*.

### Example 4. cDNA Expression Cloning of the Keratinocyte Growth Factor Receptor by Complementation for Autocrine Transformation

### 4.1 Identification of epithelial cell cDNAs capable of transforming NIH/3T3 cells.

We prepared a cDNA library from BALB/MK epidermal keratinocytes (Weissman, B.E. & Aaronson, S.A. Cell 32, 599-606 (1983)) using the automatic directional cloning (Miki, T. et al., Gene 83:137-146, (1989)) in an improved expression vector lpCEV27 (Miki, unpublished data). A library of 4.5 x 10⁶ independent clones was amplified, phage particles purified, and their DNA extracted. DNA transfection of NIH/3T3 mouse embryo fibroblasts (Jainchill, J.L. et al., J. Virol 4, 549-553 (1969)), which synthesize KGF, was performed by the calcium phosphate technique (Wigler, M. et al. Cell 11, 223-232 (1977)). Individual plates were examined at 10-18 days for the appearance of transformed foci. We detected 15 foci among a total of 100 individual cultures transfected with 5 mg library cDNA/plate. Each focus was tested and shown to be resistant to G418, indicating that it contained integrated vector sequences. Three representative transformants were chosen for more detailed characterization based upon differences in their morphologies (Fig. 13).

When we performed plasmid rescue, each transformant gave rise to at least 3 distinct cDNA clones as determined by physical mapping. To examine their biological activities, each clone was subjected to transfection analysis on NIH/3T3 cells. A single clone rescued from each transformant was found to possess high-titered transforming activity ranging from 10³-10⁴ focus forming units/nmole DNA. Moreover, the morphology of foci induced by each cDNA was similar to that of the parental transformant. Because of their distinct physical maps and distinguishable biological properties, we tentatively designated the genes for these transforming cDNAs as epithelial cell transforming (ect) 1, 2 and 3. Transfectants induced by the individual transforming plasmids were utilized in subsequent analyses.

### 4.2 Specific KGF binding by ect1-transformed cells.

Suramin is an agent known to interfere with ligand-receptor interactions including the binding of PDGF (Fleming, T.P. et al, Proc. Natn. Acad. Sci. U.S.A. 86, 8063-8067 (1989); Belsholtz, C. et al. Proc. Natn. Acad. Sci. U.S.A. 83, 6440-6444 (1986)) and KGF with their respective receptors. When an ect1 transfectant was exposed to suramin, its proliferation was markedly inhibited, associated with reversion of the transformed phenotype (data not shown). To further investigate the possibility that ect1 might encode the KGF receptor, we performed binding studies with recombinant [¹²⁵I]-KGF as the tracer molecule. As shown in Fig. 14, BALB/MK cells demonstrated specific high affinity binding of [¹²⁵I]-KGF while there was no such binding detectable to NIH/3T3 cells. Of note, expression of the ect1 gene by NIH/3T3 cells resulted in the acquisition of 3.5-fold more [¹²⁵I]-KGF binding sites than BALB/MK cells (Fig. 13). Under these same conditions, neither NIH/ect2 nor ect3 bound significant levels of the labelled growth factor. These results strongly suggested that ect1 encoded the KGF receptor, whose introduction into NIH/3T3 cells had completed an autocrine transforming loop.

### 4.3 Molecular characterization of ect1.

To characterize ect1, the transforming 4.2kb cDNA released by SalI digestion was used as a molecular probe to hybridize SalI restricted genomic DNAs of NIH/3T3 as well as NIH/3T3 transfectants containing ect1, ect2 or ect3. While the expected 4.2kb DNA fragment was detected in the ect1 transformant (Fig. 15a), neither NIH/3T3 nor the other transfectants showed evidence of a SalI fragment hybridized by the cDNA insert. These results further argued that the ect2 and ect3 represented independent transforming genes. When EcoRI was used to cleave normal mouse DNA, we observed several distinct ect1 hybridizing DNA fragments, which reflected endogenous ect1 sequences or closely related genes (Fig. 15b). Ect1 related sequences were also demonstrated in the DNAs of other species analyzed, including human, indicating its high degree of conservation in vertebrate evolution.

When we analyzed expression of transcripts related to ect1 in BALB/MK and NIH/3T3 cells, we observed a single transcript of around 4.2 kb in BALB/MK cells (Fig. 15c). Thus, our cDNA clone represented essentially the complete ect1 transcript. In NIH/3T3 cells, a transcript of comparable size was only faintly detectable under relatively stringent hybridization conditions. We estimated that its expression was several fold lower than the level of the 4.2 transcript in BALB/MK cells. Thus, if this transcript were to represent ect1 rather than a related gene, its expression was markedly lower in fibroblasts as compared to epithelial cells.

### 4.4 Ect1 encodes a transmembrane tyrosine kinase of the FGF receptor family.

We next determined the nucleotide sequence of the ect1 4.2kb cDNA insert. Analysis of the predicted amino acid sequence revealed a long open reading frame of 2235 nucleotides (nucleotide position 562-2796). Two methionine codons were found at nucleotide positions 619 and 676, respectively (Fig. 16a). The second methionine codon perfectly matched the Kozak's consensus for a translational initiator sequence (A/GCCATGG) (Kozak, M. Nucleic Acids Res. 15, 8125-8148 (1987)). Moreover, it was followed by a characteristic signal sequence of 21 residues of which 10 were identical to those of the putative signal peptide of the mouse bFGF receptor (Reid, H.H., et al., Proc. Natn. Acad. Sci U.S.A. 87, 1596-1600 (1990); Pasquale, E.B. & Singer, S.J. Proc. Natn. Acad. Sci. U.S.A. 86, 8722-8726 (1989); (Safran, A. et al. Oncogene 5, 635-643 (1990)). Thus, it seems likely that the second ATG is the authentic initiation codon for the KGF receptor (KGFR). If so, the receptor polypeptide would comprise 707 amino acids with a predicted size of 82.5 kd.

The amino acid sequence of the KGFR predicted a transmembrane tyrosine kinase most closely related to the mouse bFGF receptor (bFGFR). The percent similarity between both proteins is shown in Fig. 16b. The putative KGFR extracellular portion contained two immunoglobulin (IgG)-like domains, exhibiting 77% and 60% similarity with the IgG-like domains 2 and 3, respectively, of the mouse bFGFR. Recent studies have revealed a variant form of the bFGFR, whose extracellular domain also contains only these two corresponding IgG-like domains (Reid, H.H., et al., Proc. Natn. Acad. Sci U.S.A. 87, 1596-1600 (1990). The sequence N-terminal to the first IgG-like domain of the KGFR was 63 residues long in comparison to 88 residues found in the shorter form of the mouse bFGFR. Both the chicken and mouse bFGFRs contain a series of eight consecutive acidic residues between the first and second IgG-like domains (Reid, H.H., et al., Proc. natn. Acad. Sci U.S.A. 87, 1596-1600 (1990); Pasquale, E.B. & Singer, S.J. Proc. Natn. Acad. Sci. U.S.A. 86, 8722-8726 (1989); (Safran, A. et al. Oncogene 5, 635-643 (1990); (Lee, P.L. et al. Science 245, 57-60 (1989)). This sequence is even retained in the shorter form of the mouse bFGFR, which lacks the first IgG-like domain (Fig. 16b). However, the KGFR did not contain such an acidic domain. Whether this reflects significant functional differences between these receptors remains to be determined.

The intracellular portion of the KGFR was highly homologous to the bFGFR tyrosine kinase (Fig. 16). The central core of the catalytic domain was flanked by a relatively long juxtamembrane sequence, and the tyrosine kinase domain was split by a short insert of 14 residues, similar to that observed in avian, human and murine bFGF receptors (Reid, H.H., et al., Proc. natn. Acad. Sci U.S.A. 87, 1596-1600 (1990); Pasquale, E.B. & Singer, S.J. Proc. Natn. Acad. Sci. U.S.A. 86, 8722-8726 (1989); (Safran, A. et al. Oncogene 5, 635-643 (1990); (Lee, P.L. et al. Science 245, 57-60 (1989); (Ruta, M. et al. Oncogene 3, 9-15 (1988); and (Ruta, M. et al. Proc. Natn. Acad. Sci. U.S.A. 86, 5449-5434 (1989)). Hanafusa and cc-workers isolated a partial cDNA encoding a tyrosine kinase, designated bek, by bacterial expression cloning using phosphotyrosine antibodies (Kornbluth, S., et al., Molec. Cell Biol. 8, 5541-5544 (1988)). The reported sequence of bek was identical to the KGFR in the tyrosine kinase domain (Fig. 16b). Although only partical sequence of bek is available, it is very likely to encode the mouse KGF receptor.

### 4.5 Functional analysis of the cloned KGF receptor.

Because of the existence of more than one receptor of the FGF family (Reid, H.H., et al. Proc. Natn. Acad. Sci. U.S.A. 87, 1596-1600 (1990), we sought to characterize in detail the binding properties of the KGF receptor isolated by expression cloning. Scatchard analysis of [¹²⁵I]-KGF binding to the NIH/ect1 transfectant revealed expression of two similar high affinity receptor populations. Out of a total of ∼3.8 x 10⁵ sites/cell, 40% displayed a Kd of 180 pM, while the remaining 60% showed a Kd of 480 pM (data not shown). These values are comparable to the high affinity KGF receptors displayed by BALB/MK cells. The pattern of KGF and FGF competition for [¹²⁵I]-KGF binding to NIH/ect1 cells was also very similar to that observed with BALB/MK cells (Fig. 17). Although maximum [¹²⁵I]-KGF binding to NIH/ect1 cells was 3.5 fold higher than to BALB/MK, there was 50% displacement by 2 ng/ml of either KGF or aFGF with each cell type. Similarly, both cells showed 15-fold less efficient competition by bFGF for bound [¹²⁵I]-KGF. Together with observations that parental NIH/3T3 cells lack detectable specific [¹²⁵I]-KGF binding (Fig. 14), these results demonstrate that the cloned KGF receptor expressed in NIH/3T3 cells conferred the characteristic pattern of KGF and FGF competition displayed by BALB/MK cells.

When [¹²⁵I]-KGF is crosslinked to its receptors on BALB/MK cells, two protein species of 165 and 137kd have been observed. Taking into account the size of KGF itself, we have estimated the corresponding receptor species to be around 140 and 115kd, respectively. When [¹²⁵I]-KGF crosslinking was performed with NIH/ect1 cells, we observed a single species corresponding in size to the smaller, 137kd species in BALB/MK cells (Fig. 17a). Moreover, detection of this band was specifically and efficiently blocked by unlabelled KGF. When glycosylation is considered, the size of the KGF receptor predicted by sequence analysis corresponds reasonably well with the corrected size (115 kd) of the crosslinked KGF receptor in the ect1 transfectant.

As a final test of the functional nature of the KGF receptor expressed in NIH/ect1 cells, we investigated its capacity to induce tyrosine phosphorylation of cellular proteins. Thus, intact NIH/ect1 cells were exposed to KGF for 10 min, and cell lysates were subjected to immunoprecipitation and immunoblotting analysis utilizing anti-phosphotyrosine (anti-Ptyr) antibody. As shown in Fig. 17b, several putative substrates were tyrosine phosphorylated in response to KGF addition. These included pp55, pp65, pp90, pp115, pp150 and pp190. Previous studies have indicated that similar size proteins are phosphorylated in response to KGF triggering of BALB/MK cells. Moreover, the 115-kd phosphoprotein matches the corrected size of the KGF receptor crosslinked by [¹²⁵I]-KGF. Thus, it may reflect the autophosphorylated KGF receptor itself.

For purposes of completing the background description and present disclosure, each of the published articles, patents and patent applications heretofore identified in this specification are hereby incorporated by reference into the specification.

The foregoing invention has been described in some detail for purposes of clarity and understanding. It will also be obvious that various combinations in form and detail can be made without departing from the scope of the invention.

One skilled in the art will appreciate that the capacity for efficient rescue of cDNA clones from mammalian cells is an important function of a stable mammalian expression cloning system. When plasmid cDNA libraries are used to transfect mammalian cells, single plasmids integrated in genomic DNA are difficult to release. Plasmid rescue is readily achieved only when multiple copies are clustered at a single integration site (Noda et al, PNAS, 86, 162-166, 1989). Excision of the plasmid by induction of replication from the SV40 origin using COS cell fusion often results in rearrangement or truncation of cDNA clones efficiently from stable integration sites within mammalian host cells, Applicants used a strategy involving -plasmid composite vectors. The vectors contain plasmid excision sites for multiple cutters including *Not1*, *MLuI* and *Xhol*. This allows the intact plasmid containing insert to be efficiently rescued with low probability of internal cleavage of the insert.

## Claims

1. A DNA segment encoding a keratinocyte growth factor receptor.

2. The DNA segment according to claim 1 wherein said receptor has the amino acid sequence given in Figure 15a, or allelic or species variations thereof.

3. A DNA construct comprising a vector and a DNA segment encoding a keratinocyte growth factor receptor.

4. A DNA construct comprising a vector and a DNA segment encoding a keratinocyte growth factor receptor, wherein the vector is a genetic cloning vector comprising at least one replicon;
a site for inserting a DNA segment to be cloned that includes at least two non-symmetrical restriction enzyme recognition sequences that can be cleaved by a single restriction enzyme; and
at least two regulatory elements located in relation to said site for insertion such that, when a DNA segment is inserted into said site, transcription of said DNA segment can be effected in both the sense and antisense directions.

5. The construct according to claim 3 wherein the vector is pCEV27.

6. A host cell comprising the construct according to claim 3.

7. A keratinocyte growth factor receptor substantially free of proteins with which it is naturally associated.

8. The receptor according to claim 7 wherein said receptor has a higher affinity for keratinocyte growth factor and acidic fibroblast growth factor than basic fibroblast growth factor.

9. The receptor according to claim 8 wherein said receptor has the sequence shown in Figure 15a.

10. A process of producing a keratinocyte growth factor receptor comprising culturing the cells according to claim 6 under conditions such that said DNA segment is expressed and said factor thereby produced.

11. A method of identifying the presence in a DNA sequence of a gene the protein product of which confers a phenotypically identifiable trait comprising:
i) preparing a DNA expression library containing said DNA sequence in a cloning vector, in a manner such that said gene is represented in a said library;
ii) introducing said library into a population of cells under conditions such that integration into the genome of said cells and expression of said gene are effected, so that said phenotypically identifiable trait is caused to be displayed;
iii) isolating DNA from said cells of said population displaying said phenotypically identifiable trait; and
iv) excising a DNA segment containing said gene from said integrated DNA, wherein said cloning vector is one of the following:
a) a genetic cloning vector comprising at least one replicon; and
a site for inserting DNA segments to be cloned that includes at least two non-symmetrical restriction enzyme recognition sequences, wherein
at least two of said non-symmetrical restriction enzyme recognition sequences are nonidentical, said vector further comprising regulatory elements that are located in relation to said site for insertion of DNA segments such that, when a DNA segment is inserted into said site, at least a portion of the sequence of said DNA segment is transcribed; or
b) a genetic cloning vector comprising
at least one replicon;
a site for inserting a DNA segment to be cloned that includes at least two non-symmetrical restriction enzyme recognition sequences that can be cleaved by a single restriction enzyme; and
at least two regulatory elements located in relation to said site for insertion such that, when a DNA segment is inserted into said site, transcription of said DNA segment can be effected in both the sense and antisense directions.

12. The method according to claim 11 wherein said DNA sequence is a cDNA sequence.

13. The method according to claim 11 wherein said gene encodes a ligand, a receptor for which is normally produced by cells of said population, said cells, prior to introduction of said gene, being incapable of producing said ligand.

14. The method according to claim 11 wherein said gene encodes a receptor, the ligand which binds thereto being normally produced by cells of said population, said cells, prior to introduction of said gene, being incapable of producing said receptor.

15. The method according to claim 11 wherein said phenotypically identifiable trait is uncontrolled proliferation.

16. The method according to claim 14 wherein said receptor is the keratinocyte growth factor receptor.

17. The method according to claim 11 wherein said phenotypically identifiable trait is drug resistance.

18. A DNA segment having a sequence that encodes the amino acid sequence shown in Figure 9b.

19. A DNA segment according to claim 18 wherein said segment has the sequence shown in Figure 9b.
